# EUROPÄISCHE PATENTANMELDUNG

(11) **EP 1 815 843 A2**
(43) Veröffentlichungstag der Anmeldung: **08.08.2007**
(21) Anmeldenummer: 06025995.9
(22) Anmeldetag: 15.12.2006
(51) Int. Cl.: A61K 8/64, A61Q 19/02, A61Q 19/08

(54) **Synergistische Proteinhydrolysat-Kombinationen zur Behandlung reifer Haut**

(30) Priorität: 29.12.2005 DE 102005063062
(71) Anmelder: Henkel KGaA, 40589 Düsseldorf (DE)
(72) Erfinder: Waldmann-Laue, Marianne, 40789 Monheim (DE); Wadle, Armin, 40699 Erkrath (DE); Heinen, Soraya, 50858 Köln (DE); Jassoy, Claudia, 40237 Düsseldorf (DE); Bähren, Annika, 41363 Jüchen (DE)

(57) **Zusammenfassung**

Die Erfindung betrifft topische kosmetische oder dermatologische Zusammensetzungen zur Behandlung reifer oder intrinsisch oder extrinsisch gealterter Haut, insbesondere zur Antifaltenbehandlung, die in einem geeigneten kosmetischen oder dermatologischen Träger eine Kombination von mindestens einem Sojaproteinhydrolysat mit einem mittleren Molekulargewicht im Bereich von 1200 - 1800 Dalton, mindestens einem Sojaproteinhydrolysat mit einem mittleren Molekulargewicht im Bereich von 600 - 1000 Dalton und mindestens einem Weizenproteinhydrolysat enthalten.

## Beschreibung

Die Erfindung betrifft topische kosmetische oder dermatologische Zusammensetzungen zur Behandlung reifer oder intrinsisch oder extrinsisch gealterter oder lichtgeschädigter Haut, insbesondere zur Antifaltenbehandlung, die in einem geeigneten kosmetischen oder dermatologischen Träger eine synergistische Proteinhydrolysat-Kombination von mindestens zwei Sojaproteinhydrolysaten mit unterschiedlichen mittleren Molekulargewichtsverteilungen und mindestens einem Weizenproteinhydrolysat enthalten.

Eine maßgebliche Folge der Hautalterung ist der Verlust an Kollagen. Dieser basiert zum einen auf einer verminderten Kollagensynthese in bestimmten Hautzellen, den Fibroblasten, zum anderen auf einem verstärkten Kollagenabbau, insbesondere durch bestimmte Enzyme, wie der sogenannten Matrix-Metalloproteinase-1 (MMP-1). Insbesondere führt die UV-induzierte Expression der Matrix-Metalloproteinase-1 zu einem Abbau von Kollagen-1, das ca. 85% des totalen Proteins in der extrazellulären Matrix ausmacht. Neben den Elementen des Zytoskeletts und den Gap Junction-Proteinen vermitteln weitere Proteine dem Hautgewebe mechanische Stabilität. Diese Proteine vermitteln eine Bindung zwischen Zellen und extrazellulärer Matrix (ECM). Bestandteil der ECM ist z. B. das Kollagen oder auch die Hyaluronsäure. Für die Bindung von Zellen an die Proteine der ECM sind Rezeptoren verantwortlich, die auf der Zelloberfläche lokalisiert sind. So bindet z.B. der Oberflächenrezeptor CD44, dessen Expression beispielsweise durch Apfelkernextrakte gesteigert wird, an Hyaluronsäure.
Neuere Untersuchungen haben die Annahme erhärtet, dass durch den Kollagenabbau das Integrin-vermittelte Wechselspiel zwischen Kollagenmatrix und Fibroblasten gestört wird. Es wurde gezeigt, dass auf einer vorgeschädigten, kontrahierten und spannungsarmen Kollagenmatrix die weitere Kollagensynthese nur eingeschränkt erfolgt. Der Verlust an mechanischer Spannung scheint kausal an der verminderten Neusynthese von Pro-Kollagen beteiligt zu sein (J. Varani et al., Reduced Fibroblast Interaction with Intact Collagen as a mechanism for depressed Collagen Synthesis in Photodamaged Skin, J. Invest. Dermatol. 122, 1471 - 1479, 2004).

Es besteht ein großes Interesse an wirksamen kosmetischen Produkten, die in der Lage sind, den altersbedingten Verlust an Kollagen nachhaltig auszugleichen, um so das Erscheinungsbild der alternden Haut zu verbessern. Kosmetische Anti-Ageing Produkte enthalten daher oftmals Wirkstoffe, die in der Lage sind, die Kollagensynthese zu stimulieren.

Topische Zusammensetzungen, die Peptide enthalten, die die Kollagensynthese stimulieren, sind im Stand der Technik bekannt. Derartige Zusammensetzungen zeigen eine gewisse Antifaltenleistung, die im Ergebnis jedoch noch nicht befriedigend ist. Topische Hautpflegeprodukte mit nachgewiesener Antifaltenleistung, die z. B. Tretinoin oder α-Hydroxycarbonsäure enthalten, sind häufig aufgrund der notwendigen höheren Konzentrationen dieser Wirkstoffe nur mäßig oder sogar schlecht hautverträglich. Andere Wirkstoffe mit nachgewiesener Antifaltenleistung sind instabil z.B. gegenüber chemischem Abbau, z. B. Tretinoin oder Flavonoide. Phytoflavone, die ebenfalls eine Antifaltenleistung aufweisen, können hormonell wirken und sind daher für Kosmetika weniger bevorzugt.
Dagegen mangelt es im Stand der Technik an wirksamen Zubereitungen, die in der Lage sind, vorhandene alterungsbedingte Defizite insbesondere im Bereich der extrazellulären Matrix zu reparieren.

Eine Aufgabe der vorliegenden Erfindung war es, topische kosmetische oder dermatologische Zusammensetzungen zur Behandlung reifer oder intrinsisch oder extrinsisch gealterter Haut, insbesondere zur Antifaltenbehandlung, mit einer gegenüber dem Stand der Technik optimierten Wirksamkeit bereitzustellen. Eine weitere Aufgabe war es, topische kosmetische oder dermatologische Zusammensetzungen zur Behandlung lichtgeschädigter oder reifer oder intrinsisch oder extrinsisch gealterter Haut, insbesondere zur Antifaltenbehandlung, mit einer optimierten Hautverträglichkeit bereitzustellen.

Überraschend und für den Fachmann nicht vorhersehbar wurde nun festgestellt, dass mit Zusammensetzungen zur topischen Behandlung der Haut, die in einem geeigneten kosmetischen oder dermatologischen Träger mindestens ein Sojaproteinhydrolysat mit einem mittleren Molekulargewicht im Bereich von 1200 - 1800 Dalton, bevorzugt im Bereich von 1400 - 1700 Dalton, mindestens ein Sojaproteinhydrolysat mit einem mittleren Molekulargewicht im Bereich von 600 - 1000 Dalton, bevorzugt 800 Dalton, und mindestens ein Weizenproteinhydrolysat enthalten, die Nachteile des Standes der Technik beseitigt werden und Zusammensetzungen zur Behandlung reifer oder intrinsisch oder extrinsisch gealterter Haut, insbesondere zur Antifaltenbehandlung, erhalten werden, deren Effekte auf die Faltenglättung und die Verbesserung des Hauterscheinungsbildes gegenüber den Zusammensetzungen des Standes der Technik in synergistischer Weise gesteigert und verbessert werden. Gleichzeitig wurde gefunden, dass mit Zusammensetzungen, die in einem geeigneten kosmetischen oder dermatologischen Träger mindestens ein Sojaproteinhydrolysat mit einem mittleren Molekulargewicht im Bereich von 1200 - 1800 Dalton, bevorzugt im Bereich von 1400 - 1700 Dalton, mindestens ein Sojaproteinhydrolysat mit einem mittleren Molekulargewicht im Bereich von 600 - 1000 Dalton, bevorzugt 800 Dalton, und mindestens ein Weizenproteinhydrolysat enthalten, eine wirksame Antifaltenbehandlung mit verbesserter Hautverträglichkeit erfolgen kann.

Gegenstand der vorliegenden Erfindung sind kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten.

Ein erfindungsgemäß besonders bevorzugtes Sojaproteinhydrolysat mit einem mittleren Molekulargewicht (Mw) im Bereich von 1200 - 1800 Dalton, bevorzugt im Bereich von 1400 - 1700 Dalton (Da), ist unter dem Handelsnamen Ridulisse C von der Firma Silab erhältlich. Der Proteinhydrolysat-Anteil in Ridulisse C enthält vier Molekulargewichtsfraktionen: 0,8 Gew.-% mit einem Mw > 12.500 Da, 22,2 Gew.-% mit 1355 Da < Mw < 12.500 Da, 43,1 Gew.-% 75 Da < Mw < 1355 Da und 33,9 Gew.-% < 75 Da.
Ridulisse C erhöht und/oder verbessert die Interaktion zwischen der extrazellulären Matrix und den Fibroblasten. Erfindungsgemäß wird unter der Interaktion zwischen der extrazellulären Matrix (ECM) und den Fibroblasten sowohl die Interaktion zwischen dem Kollagen der ECM und den Fibroblasten als auch die Interaktion zwischen der Hyaluronsäure der ECM und den Fibroblasten verstanden.

Erfindungsgemäß besonders bevorzugte kosmetische oder dermatologische Zusammensetzungen sind dadurch gekennzeichnet, dass sie mindestens ein Sojaproteinhydrolysat mit einem mittleren Molekulargewicht (Mw) im Bereich von 1200 - 1800 Dalton, bevorzugt im Bereich von 1400 - 1700 Dalton, in einer Gesamtmenge von 0,0001 - 5 Gew.-%, bevorzugt 0,001 - 1 Gew.- %, besonders bevorzugt 0,01 - 0,1 Gew.-% und außerordentlich bevorzugt 0,02 - 0,05 Gew.-%, enthalten, jeweils bezogen auf den Gehalt an Aktivsubstanz in der gesamten Zusammensetzung.

Ein erfindungsgemäß besonders bevorzugtes Sojaproteinhydrolysat mit einem mittleren Molekulargewicht im Bereich von 600 - 1000 Dalton, bevorzugt 800 Dalton, ist unter dem Handelsnamen Phytokine von der Firma Coletica erhältlich. Es stimuliert die Kollagensynthese.

Erfindungsgemäß besonders bevorzugte kosmetische oder dermatologische Zusammensetzungen sind dadurch gekennzeichnet, dass sie mindestens ein Sojaproteinhydrolysat mit einem mittleren Molekulargewicht (Mw) im Bereich von 600 - 1000 Dalton, bevorzugt 800 Dalton, in einer Gesamtmenge von 0,0001 - 1 Gew.-%, bevorzugt 0,001 - 0,1 Gew.-%, besonders bevorzugt 0,005 - 0,05 Gew.-% und außerordentlich bevorzugt 0,006 - 0,01 Gew.-%, enthalten, jeweils bezogen auf den Gehalt an Aktivsubstanz in der gesamten Zusammensetzung.

Ein erfindungsgemäß besonders bevorzugtes Weizenproteinhydrolysat ist unter dem Handelsnamen Liftiline von der Firma Silab erhältlich.

Erfindungsgemäß besonders bevorzugte kosmetische oder dermatologische Zusammensetzungen sind dadurch gekennzeichnet, dass sie mindestens ein Weizenproteinhydrolysat in einer Gesamtmenge von 0,001 - 5,0 Gew.-%, bevorzugt 0,01 - 1,0 Gew.-%, besonders bevorzugt 0,1 - 0,5 Gew.-% und außerordentlich bevorzugt 0,2 - 0,4 Gew.-%, enthalten, jeweils bezogen auf den Gehalt an Aktivsubstanz in der gesamten Zusammensetzung.

Erfindungsgemäß besonders bevorzugte kosmetische oder dermatologische Zusammensetzungen sind dadurch gekennzeichnet, dass sie a) mindestens ein Sojaproteinhydrolysat mit einem mittleren Molekulargewicht im Bereich von 1200 - 1800 Dalton, bevorzugt im Bereich von 1400 - 1700 Dalton b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat in Gewichtsverhältnissen von 1 : (0,1-1): (1-50), bevorzugt 1 : (0,2-0,6): (10-20), enthalten.
Besonders überraschend wurde beobachtet, dass die positiven Effekte der Dreierkombination a) bis c) deutlich schneller einsetzen als bei einer Behandlung mit nur einem oder zwei der Wirkstoffen. Bereits nach einmaliger Behandlung wurden deutliche Verbesserungen in der Hautstruktur und dem Erscheinungsbild der Haut beobachtet.

Erfindungsgemäß besonders bevorzugte kosmetische oder dermatologische Zusammensetzungen sind dadurch gekennzeichnet, dass sie zusätzlich zu der Dreierkombination aus den Proteinhydrolysaten a) bis c) mindestens einen weiteren, die Kollagensynthese stimulierenden Wirkstoff enthalten, der ausgewählt ist aus
- Hydroxyprolin, das mit zwei, bevorzugt linearen, C₂-C₂₂-Fettsäureresten N-acyliert und verestert ist, insbesondere Dipalmitoylhydroxyprolin,
- den Tripeptiden Gly-His-Lys, Lys-Val-Lys, Lys-Val-Dab, Lys-Phe-Lys, Lys-Ile-Lys, Dab-Val-Lys, Lys-Val-Orn, Lys-Val-Dap, Dap-Val-Lys und Gly-His-Arg,
- den Tetrapeptiden Gly-Gln-Pro-Arg (Rigin), Gly-Gln-Arg-Pro, Val-Val-Arg-Pro, Rigin-Analoga sowie ALAMCAT-Tetrapeptiden,
- dem Pentapeptid Lys-Thr-Thr-Lys-Ser,
- den Hexapeptiden Val-Gly-Val-Ala-Pro-Gly, Ala-Asp-Leu-Lys-Pro-Thr (Hexapeptide-3), Hexapeptide-4, Hexapeptide-5, Hexapeptide-6, Hexapeptide-8, Hexapeptide-9 und Hexapeptide-10,
- den mit mindestens einer, bevorzugt linearen, C₂-C₂₂-Fettsäure N-acylierten und/oder veresterten Derivaten der genannten Tri-, Tetra-, Penta- und Hexapeptide, insbesondere N-Palmitoyl-Gly-His-Lys, N-Palmitoyl-Lys-Val-Lys, N-Myristoyl-Gly-His-Arg, N-Palmitoyl-Gly-Gln-Pro-Arg, N-Palmitoyl-Lys-Thr-Thr-Lys-Ser, N-Palmitoyl-Val-Gly-Val-Ala-Pro-Gly, Acetyl-Hexapeptide-3, Myristoyl Hexapeptide-5, Myristoyl Hexapeptide-8 sowie Mischungen aus N-Palmitoyl-Gly-His-Lys und N-Palmitoyl-Gly-Gln-Pro-Arg,
- Retinoiden, insbesondere Retinol und C₂-C₂₂-Fettsäureestern des Retinols,
- Saccharomyces/Xylinum/Black Tea Ferment,
- Olivenblattextrakten (Olea Europaea (Olive) Leaf Extract),
- Oleanolsäure,
- Oleanol,
- Extrakten aus Haferkörnern (Avena Sativa (Oat) Kernel Extract),
- liposomenverkapselten Grüntee-Extrakten (Camellia Sinensis),
- Kreatin,
- sowie Mischungen der vorgenannten Substanzen.

Mit einem Gehalt an mindestens einem weiteren der vorstehenden die Kollagensynthese stimulierenden Wirkstoffe wird das äußere Erscheinungsbild der Haut noch weiter verbessert. Insbesondere kann so der Feuchtigkeitsgehalt der Haut gesteigert werden.

Es kann erfindungsgemäß besonders bevorzugt sein, dass die als Wirkstoff eingesetzten Aminosäuren und Peptide zur Verbesserung der Penetration in die Haut mit mindestens einer, bevorzugt linearen, C₂-C₂₂-Fettsäure N-acyliert und/oder verestert sind. Zur N-Acylierung und/oder Veresterung besonders bevorzugt sind C₈-C₁₈-Fettsäuren, ganz besonders bevorzugt sind Myristinsäure (C₁₄) und Palmitinsäure (C₁₆). Besonders bevorzugt ist weiterhin, dass alle verwendeten Aminosäuren und Peptide derartig N-acyliert und/oder verestert sind. Ebenfalls bevorzugt ist die Substitution der Aminosäuren und Peptide mit einer Benzyloxycarbonylgruppe an der terminalen Aminogruppe.

Erfindungsgemäß bevorzugte zusätzliche Wirkstoffe, die die Kollagensynthese stimulieren, sind ausgewählt aus Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift PDHP von der Firma Seppic erhältlich ist.

Ein erfindungsgemäß bevorzugtes Peptid, das die Kollagensynthese stimuliert, ist das Tripeptid Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können bevorzugt Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und lle bevorzugt geeignet. Die erfindungsgemäß besonders bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn oder Citrullin ersetzt. Ein weiteres erfindungsgemäß bevorzugtes Peptid, das die Kollagensynthese stimuliert, ist dementsprechend Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist.
Weitere erfindungsgemäß bevorzugte Tripeptide, die die Kollagensynthese stimulieren, sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure) und Dap-Val-Lys. Ein besonders bevorzugtes Tripeptid ist Palmitoyl-Lys-Val-Lys, z. B. erhältlich von der Firma Pentapharm unter der Bezeichnung SYN^{®}-COLL.

Weitere erfindungsgemäß bevorzugte Tetrapeptide, die die Kollagensynthese stimulieren, sind das Tetrapeptid Rigin und Rigin-basierte Tetrapeptide sowie ALAMCAT-Tetrapeptide. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg (INCI-Bezeichnung: Palmitoyl Tetrapeptide-1), das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapeptide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können. Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (IIe).
Die bevorzugten Aminosäuren, die Gln ersetzen können, beinhalten eine Seitenkette mit einer Aminogruppe, die bei neutralem pH (pH 6-7) überwiegend ungeladen vorliegt, z. B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.

Weitere erfindungsgemäß bevorzugte Peptide, die die Kollagensynthese stimulieren, sind das Pentapeptid Lys-Thr-Thr-Lys-Ser und seine N-acylierten Derivate, besonders bevorzugt N-Palmitoyl-Lys-Thr-Thr-Lys-Ser, das unter der Bezeichnung Matrixyl von der Firma Sederma erhältlich ist.

Weitere erfindungsgemäß bevorzugte Hexapeptide, die die Kollagensynthese stimulieren, sind das Hexapeptid Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist. Weitere erfindungsgemäß bevorzugte Peptide sind die Hexapeptide und/oder deren N-acylierten Derivate, Ala-Asp-Leu-Lys-Pro-Thr (Hexapeptide-3, z. B. Peptide 02 von Vincience), Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec).

Weitere erfindungsgemäß bevorzugte Wirkstoffe, die die Kollagensynthese stimulieren, sind ausgewählt aus Retinoiden. Zu den Retinoiden werden im Sinne der vorliegenden Erfindung Retinol (Vitamin A₁), die C₂-C₂₂-Fettsäureester des Retinols (= Retinylester, insbesondere Retinylpalmitat und Retinylacetat), 3,4-Didehydroretinol (Vitamin A₂), Retinal und seine Isomere, *all-trans-*Retinsäure, 9-*cis*-Retinsäure und 13-*cis*-Retinsäure (Tretinoin), die Ester der Retinsäure sowie weitere verwandte Substanzen (synthetische Retinoide) gezählt, die für ihre vielfältige biologische Wirkung insbesondere auf Wachstum und Differenzierung bekannt sind, sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen. Synthetische Retinoide werden in drei Gruppen eingeteilt: nichtaromatisch (z. B. *Isotretinoin),* monoaromatisch (z.B. Acitretin) und polyaromatisch (sogenannte Arotinoide, z. B. Tazarotene, die eine spezifische Wirkung auf einzelne Retinoid-Rezeptoren haben). Erfindungsgemäß besonders bevorzugte Retinoide sind Retinol und die C₂-C₂₂-Fettsäureester des Retinols, insbesondere Retinylpalmitat und Retinylacetat.

Weitere erfindungsgemäß bevorzugte Wirkstoffe, die die Kollagensynthese stimulieren, sind ausgewählt aus Produkten, die durch Fermentation von gezuckertem Schwarztee mit den zwei symbiotischen Mikroorganismen Saccharomyces und Acetobacter Xylinum gewonnen werden und die INCI-Bezeichnung Saccharomyces/Xylinum/Black Tea Ferment tragen. Ein besonders bevorzugtes Produkt ist unter dem Handelsnamen Kombuchka von der Firma Sederma erhältlich (INCI-Bezeichnung: Saccharomyces/Xylinum/Black Tea Ferment, Glycerin, Hydroxyethylcellulose).

Weitere erfindungsgemäß bevorzugte Wirkstoffe, die die Kollagensynthese stimulieren, sind ausgewählt aus Olivenblattextrakten (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.

Olivenblattextrakte können einen hohen Gehalt an Oleanolsäure und/oder Oleanol aufweisen. Weitere erfindungsgemäß bevorzugte Wirkstoffe, die die Kollagensynthese stimulieren, sind ausgewählt aus Oleanolsäure und Oleanol.

Weitere erfindungsgemäß bevorzugte Wirkstoffe, die die Kollagensynthese stimulieren, sind ausgewählt aus Extrakten aus Haferkörnern (Avena Sativa (Oat) Kernel Extract). Ein erfindungsgemäß besonders bevorzugter Extrakt aus Haferkörnern ist unter der Handelsbezeichnung Drago Beta Glucan (02/060800) von der Firma Symrise erhältlich.

Weitere erfindungsgemäß bevorzugte Wirkstoffe, die die Kollagensynthese stimulieren, sind ausgewählt aus liposomenverkapselten Grüntee-Extrakten (Camellia Sinensis). Ein erfindungsgemäß besonders bevorzugter liposomenverkapselter Grüntee-Extrakt ist unter der Handelsbezeichnung Greenselect Phytosome von der Firma Indena erhältlich.

Erfindungsgemäß besonders bevorzugte kosmetische oder dermatologische Zusammensetzungen sind dadurch gekennzeichnet, dass sie zusätzlich zu der Dreierkombination aus den Proteinhydrolysaten a) bis c) mindestens einen Wirkstoff, der die Kollagensynthese stimuliert, in einer Gesamtmenge von 0,000001 - 5 Gew.-%, bevorzugt 0,00001 - 2 Gew.-%, besonders bevorzugt 0,0001 - 1 Gew.-% und außerordentlich bevorzugt 0,005 - 0,5 Gew.-%, enthalten, jeweils bezogen auf den Gehalt an Aktivsubstanz in der gesamten Zusammensetzung.

Weitere erfindungsgemäß besonders bevorzugte kosmetische oder dermatologische Zusammensetzungen sind dadurch gekennzeichnet, dass sie zusätzlich zu der Dreierkombination aus den Proteinhydrolysaten a) bis c) mindestens einen Wirkstoff enthalten, der die Interaktion zwischen der extrazellulären Matrix und den Fibroblasten erhöht und/oder verbessert und der ausgewählt ist aus
- mit Kokosfettsäuren N-acylierten und/oder veresterten Sojaproteinhydrolysaten in Form ihrer Alkalimetallsalze, bevorzugt in Form der Kaliumsalze,
- Ascorbinsäure, den Estern der Ascorbinsäure mit anorganischen und/oder organischen Säuren, den Ethern der Ascorbinsäure mit Mono-, Oligo- und Polysacchariden sowie den physiologisch verträglichen Salzen dieser Komponenten,
- Apfelkernextrakten (Pyrus Malus (Apple) Fruit Extract),
- Lotuskeim-Extrakten (Nelumbo Nucifera Germ Extract),
- Extrakten aus Maiskörnern (Zea Mays (Corn) Kernel Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Mischungen aus mindestens einem Extrakt aus Kakaobohnen (Theobroma cacao) und mindestens einem Extrakt aus den Blättern der Pfefferminze (Mentha piperita),
- Hydroxystilbenen und deren Estern, insbesondere Resveratrol und/oder Resveratrolmono-, -di- und -triphosphorsäureestern und deren Salzen,
- Keratinhydrolysaten, insbesondere Wollkeratinhydrolysaten,
- Conchiolinhydrolysaten,
- dem Dipeptid L-Citrullyl-L-arginin und seinen mit mindestens einer, bevorzugt linearen, C₂-C₂₂-Fettsäure N-acylierten und/oder veresterten Derivaten, bevorzugt Acetyl Citrull Amido Arginine,
- Carnitin,
- Hypotaurin,
- L-Glutamylaminoethyl-indol,
- Isoflavonoiden und Isoflavonoid-reichen Pflanzenextrakten,
- Dihydroquercetin (= Taxifolin),
- sowie Mischungen hiervon.

Mit einem Gehalt an mindestens einem weiteren der vorstehenden Wirkstoffe, die die Interaktion zwischen der extrazellulären Matrix und den Fibroblasten erhöhen und/oder verbessern, wird das äußere Erscheinungsbild der Haut noch weiter verbessert. Insbesondere kann so die Nährstoffversorgung der Hautzellen gesteigert werden, so dass weitere Wirkstoffe besser in die Haut aufgenommen werden und eine verbesserte Wirksamkeit zeigen.

Eine *in vivo*-Messung der mechanischen Spannung der ECM ist nur schwierig zu realisieren. In der vorliegenden Erfindung wurde die Interaktion zwischen der extrazellulären Matrix und den Fibroblasten a) mit Hilfe eines aus nativem Kollagen Typ I und humanen Vorhaut-Fibroblasten gebildeten dreidimensionalen kontraktionsfähigen Kollagengels oder b) gemäß der in der Offenlegungsschrift WO 2003/053394 A2 dargelegten Steigerung der Expression des Oberflächenrezeptors CD44 bestimmt.
Bei Methode a) wurde die Fibroblasten-vermittelte Gelkontraktion durch eine zeitabhängige Veränderung der Gelfläche nach Behandlung mit dem zu untersuchenden Wirkstoff beobachtet. Die Kontraktion des Kollagengels wurde als Interaktion zwischen den Fibroblasten und der Kollagenmatrix interpretiert: je stärker die Interaktion zwischen den Fibroblasten und der Kollagenmatrix ist, desto stärker und/oder schneller zieht sich das Kollagengel unter dem Einfluss des Testwirkstoffs zusammen. Übertragen auf in vivo-Bedingungen, bedeutet das, dass die Kollagen-haltige ECM bei einer Verstärkung der Interaktion zwischen den Fibroblasten und der Kollagenmatrix unter stärkerer mechanischer Spannung stehen würde.

Weitere erfindungsgemäß bevorzugte zusätzliche Wirkstoffe, die die Interaktion zwischen der extrazellulären Matrix und den Fibroblasten erhöhen und/oder verbessern, sind ausgewählt aus mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.
Ein erfindungsgemäß besonders bevorzugtes, mit Kokosfettsäuren N-acyliertes und/oder verestertes Sojaproteinhydrolysat in Form des Kaliumsalzes ist unter der Handelsbezeichnung Coccopolipeptide di Soja von der Firma Sinerga erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Ascorbinsäure, den Estern der Ascorbinsäure mit anorganischen und/oder organischen Säuren, den Ethern der Ascorbinsäure mit Mono-, Oligo- und Polysacchariden, sowie den physiologisch verträglichen Salze dieser Komponenten. Bei den physiologisch verträglichen Salzen sind insbesondere die Zink-, Kupfer- und Mangansalze und die Salze der Alkali- und der Erdalkalimetalle bevorzugt, besonders die Natrium-, Kalium-, Magnesium- und Calcium-Salze. Erfindungsgemäß besonders bevorzugte kosmetische oder dermatologische Zusammensetzungen sind dadurch gekennzeichnet, dass sie mindestens einen Wirkstoff enthalten, der die Interaktion zwischen der extrazellulären Matrix und den Fibroblasten erhöht und/oder verbessert und der ausgewählt ist aus Ascorbinsäure und den Ascorbinsäurederivaten Natriumascorbylphosphat, Magnesiumascorbylphosphat, Ascorbylpalmitat, Dinatriumascorbylphosphat, Dinatriumascorbylsulfat, Natriumascorbat, Magnesiumascorbat, Ascorbylstearat, Ascorbyldipalmitat, Ascorbylacetat, Kaliumascorbyltocopherylphosphat, Chitosanascorbat und Ascorbylglucosid.

Weitere erfindungsgemäß bevorzugte Wirkstoffe, die die Interaktion zwischen der extrazellulären Matrix und den Fibroblasten erhöhen und/oder verbessern, sind ausgewählt aus Apfelkernextrakten (Pyrus Malus (Apple) Fruit Extract). Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.

Erfindungsgemäß besonders bevorzugte kosmetische oder dermatologische Zusammensetzungen sind dadurch gekennzeichnet, dass sie den Rohstoff Ederline in Mengen von 0,1 - 10 Gew.- %, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Apfelkernextrakt in Mengen von 0,001 - 2 Gew.-%, bevorzugt 0,01 - 1,6 Gew.-% und besonders bevorzugt 0,03 - 1 Gew.-%, jeweils bezogen auf den Gehalt an Aktivsubstanz in der gesamten Zusammensetzung, enthalten.

Weitere erfindungsgemäß bevorzugte Wirkstoffe, die die Interaktion zwischen der extrazellulären Matrix und den Fibroblasten erhöhen und/oder verbessern, sind ausgewählt aus Lotuskeim-Extrakten. Ein erfindungsgemäß besonders bevorzugter Lotuskeim-Extrakt ist unter der Handelsbezeichnung Lotus Germ Extract mit der INCI-Bezeichnung Water, Butylene Glycol, Nelumbo Nucifera Germ Extract von der Firma Maruzen erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus dem Dipeptid L-Citrullyl-L-arginin und seinen mit mindestens einer, bevorzugt linearen, C₂-C₂₂-Fettsäure N-acylierten und/oder veresterten Derivaten, insbesondere dem N-acetylierten Dipeptidderivat mit der INCI-Bezeichnung Acetyl Citrull Amido Arginine. Eine erfindungsgemäß besonders bevorzugte Zubereitung von Acetyl Citrull Amido Arginine ist unter der Handelsbezeichnung Exsy-Algine von der Firma Exsymol erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Rotweinextrakten (Wine Extract). Ein erfindungsgemäß besonders bevorzugter Rotweinextrakt ist unter der Handelsbezeichnung Sepivinol R von der Firma Seppic erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Traubenkernextrakten (Vitis Vinifera (Grape) Seed Extract), die besonders bevorzugt aus der Chardonnay-Traube stammen. Erfindungsgemäß besonders bevorzugte Traubenkernextrakte sind unter der Handelsbezeichnung Herbalia Grape von der Firma Cognis oder unter der Handelsbezeichnung Crodarom Chardonnay von Croda erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Schwarzen Holunderblüten (Sambucus Nigra Flower Extract). Ein erfindungsgemäß besonders bevorzugter Extrakt aus Schwarzen Holunderblüten ist unter der Handelsbezeichnung Sambucus AO von der Firma Alpaflor/Centerchem bzw. von Permcos erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Mischungen aus mindestens einem Extrakt aus Kakaobohnen (Theobroma cacao) und mindestens einem Extrakt aus den Blättern der Pfefferminze (Mentha piperita). Erfindungsgemäß besonders bevorzugte Mischungen aus mindestens einem Extrakt aus Kakaobohnen (Theobroma cacao) und mindestens einem Extrakt aus den Blättern der Pfefferminze (Mentha piperita) - wobei wässrige, glycolische oder wässrig-glycolische Zubereitungen dieser Extraktmischungen besonders bevorzugt sind - sind unter den Handelsnamen Caomint, Caophenol, Caobromine, Caospice und Caoorange von Solabia erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Hydroxystilbenen und deren Estern, insbesondere Resveratrol (trans-Stilben-3,4'-5-triol) und/oder Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Weitere erfindungsgemäß bevorzugte Wirkstoffe, die die Interaktion zwischen der extrazellulären Matrix und den Fibroblasten erhöhen und/oder verbessern, sind ausgewählt aus Keratinhydrolysaten, insbesondere Wollkeratinhydrolysaten. Ein erfindungsgemäß besonders bevorzugtes Wollkeratinhydrolysat ist unter der Bezeichnung Keratec Pep von der Firma Croda erhältlich. Keratec Pep weist eine kleinere Molekulargewichtsfraktion mit einem durchschnittlichen Molekulargewicht von 150 Dalton und eine größere Molekulargewichtsfraktion mit einem durchschnittlichen Molekulargewicht von 1265 Dalton auf.

Weitere erfindungsgemäß bevorzugte Wirkstoffe, die die Interaktion zwischen der extrazellulären Matrix und den Fibroblasten erhöhen und/oder verbessern, sind ausgewählt aus Conchiolinhydrolysaten. Erfindungsgemäß besonders bevorzugte Conchiolinhydrolysate sind unter den Bezeichnungen Pearl Protein Extract und Pearl Protein Extract BG von der Firma Maruzen erhältlich. Bei Conchiolinhydrolysaten handelt es sich, im Unterschied zu anderen auf dem Markt erhältlichen Perlenextrakten, nicht um Extrakte aus gemahlenen Perlen, wie sie z. B. unter der Bezeichung Crodarom Pearl mit der INCI-Bezeichnung Aqua (Water), Glycerin, Pearl Powder, Maris Sal (Sea Salt) von Croda erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet.

Ein weiterer erfindungsgemäß bevorzugter Wirkstoff, der die Interaktion zwischen der extrazellulären Matrix und den Fibroblasten erhöht und/oder verbessert, ist das Peptidderivat L-Glutamylaminoethyl-indol (erhältlich z. B. unter dem Handelsnamen Glistin von der Firma Exsymol).

Weitere erfindungsgemäß bevorzugte Wirkstoffe, die die Interaktion zwischen der extrazellulären Matrix und den Fibroblasten erhöhen und/oder verbessern, sind ausgewählt aus Isoflavonoiden und Isoflavonoid-reichen Pflanzenextrakten.
Zu den Isoflavonoiden zählen erfindungsgemäß die Isoflavone und die Isoflavon-Glycoside.
Unter Isoflavonen sind erfindungsgemäß Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Besonders bevorzugte Isoflavone sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Weiterhin ist es erfindungsgemäß bevorzugt, wenn die lsoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Lipobelle Soyaglycone (Mibelle AG Cosmetics), Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß besonders bevorzugte kosmetische oder dermatologische Zusammensetzungen sind dadurch gekennzeichnet, dass sie mindestens ein Isoflavonoid in Gesamtmengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

Ein weiterer erfindungsgemäß bevorzugter Wirkstoff, der die Interaktion zwischen der extrazellulären Matrix und den Fibroblasten erhöht und/oder verbessert, ist Dihydroquercetin (3,3',4',5,7-Pentahydroxyflavanon), das auch als Taxifolin bezeichnet wird.

Erfindungsgemäß besonders bevorzugte kosmetische oder dermatologische Zusammensetzungen sind dadurch gekennzeichnet, dass sie mindestens einen Wirkstoff, der die Interaktion zwischen der extrazellulären Matrix und den Fibroblasten erhöht und/oder verbessert, in einer Gesamtmenge von 0,000001 - 10 Gew.-%, bevorzugt 0,00001 - 5 Gew.%, besonders bevorzugt 0,0001 - 2 Gew.-% und außerordentlich bevorzugt 0,005 - 0,5 oder 1 Gew.-%, enthalten, jeweils bezogen auf den Gehalt an Aktivsubstanz in der gesamten Zusammensetzung.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen kosmetischen Wirkstoff enthalten, der ausgewählt ist aus
- Monomeren und Oligomeren von Aminosäuren, N-C₁-C₂₄-Acylaminosäuren, den Estern und/oder den physiologisch verträglichen Metallsalzen dieser Substanzen, die nicht die Kollagensynthese stimulieren und nicht die Interaktion zwischen der extrazellulären Matrix und den Fibroblasten erhöhen und/oder verbessern,
- DNA-Reparaturenzymen,
- DNA- oder RNA-Oligonucleotiden,
- Vitaminen, Provitaminen und Vitaminvorstufen der Gruppen B, E, H und K und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten, die nicht die Kollagensynthese stimulieren und nicht die Interaktion zwischen der extrazellulären Matrix und den Fibroblasten erhöhen und/oder verbessern,
- Ubichinon und Ubichinol sowie deren Derivaten,
- Silymarin,
- Ectoin,
- anorganischen und organischen UV-Filtersubstanzen,
- selbstbräunenden,
- hautberuhigenden,
- feuchtigkeitsspendenden und
- sebumregulierenden Wirkstoffen sowie
- Mischungen dieser Wirkstoffe.

Es wurde beobachtet, dass derartige Wirkstoffe auf der durch die erfindungsgemäße Proteinhydrolysat-Kombination a) bis c) optimierten Haut eine gesteigerte Wirksamkeit aufweisen.

Die Monomere der Aminosäuren und/oder der N-C₂-C₂₄-Acylaminosäuren, die nicht die Kollagensynthese stimulieren und nicht die Interaktion zwischen der extrazellulären Matrix und den Fibroblasten erhöhen und/oder verbessern, sind bevorzugt ausgewählt aus Alanin, Arginin, Asparagin, Asparaginsäure, Canavanin, Citrullin, Cystein, Cystin, Desmosin, Glutamin, Glutaminsäure, Glycin, Histidin, Homophenylalanin, Hydroxylysin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Taurin, Zink- und Natriumpyroglutamat und Natriumlauroylglutamat.
Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.

Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Die Oligomere der Aminosäuren und/oder der N-C₂-C₂₄-Acylaminosäuren, die nicht die Kollagensynthese stimulieren und nicht die Interaktion zwischen der extrazellulären Matrix und den Fibroblasten erhöhen und/oder verbessern, sind bevorzugt ausgewählt aus Di-, Tri-, Tetra-, Penta-, Hexa- oder Pentadecapeptiden, die N-acyliert und/oder verestert sein können. Erfindungsgemäß bevorzugte, gegebenenfalls N-acylierte und/oder veresterte Dipeptide sind Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß besonders bevorzugte, gegebenenfalls N-acylierte und/oder veresterte Tripeptide sind Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn und N-Acetyl-Arg-Lys-Arg-NH₂. Erfindungsgemäß bevorzugte, gegebenenfalls N-acylierte und/oder veresterte Pentapeptide N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Val-Val-Arg-Pro-Pro-Pro, Ala-Arg-His-Methylnorleucin-Homophenylalanin-Trp (Hexapeptide-7) und Hexapeptide-11 (z. B. Peptamide-6 von Arch Personal Care). Ein erfindungsgemäß besonders bevorzugtes Pentadecapeptid ist z. B. der Rohstoff Vinci 01 von Vincience (Pentadecapeptide-1).

Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.
Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.

Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosomes^{™} (INCI-Bezeichnung: Water, Lecithine, Plankton Extract) mit einer Proteinkonzentration von 0,25 - 2 mg/ml, liposomenverkapselte T4N5 z. B. unter der Bezeichnung Ultrasomes^{™} (INCI-Bezeichnung: Water, Lecithine, Micrococcus Luteus Extract) mit einer Proteinkonzentration von 2-5 mg/ml (gemessen mit dem Bradford-Assay) von AGI Dermatics, USA, erhältlich.
Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200- 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen kosmetischen Wirkstoff, der ausgewählt ist aus Photosomes^{™} und/oder Ultrasomes^{™}, in Gesamtmengen von 0,1 - 10 Gew.- %, bevorzugt 0,5 - 5,0 Gew.-% und besonders bevorzugt 1,0 - 4,0 Gew.-%, bezogen auf das gesamte Mittel, enthalten.
Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600-1000 Dalton, bevorzugt 800 Dalton, und c) mindestens ein Weizenproteinhydrolysat enthalten, sind dadurch gekennzeichnet, dass sie weiterhin mindestens einen kosmetischen Wirkstoff, der ausgewählt ist aus den Monomeren und Oligomeren von Aminosäuren, N-C₂-C₂₄-Acylaminosäuren und/oder den Estern und/oder den physiologisch verträglichen Metallsalzen dieser Substanzen, die nicht die Kollagensynthese stimulieren und nicht die Interaktion zwischen der extrazellulären Matrix und den Fibroblasten erhöhen und/oder verbessern, in Gesamtmengen von 0,00001 - 10 Gew.-%, bevorzugt 0,001 - 5 Gew.-% und besonders bevorzugt 0,005 - 3 Gew.-%, jeweils bezogen auf die gesamte Zusammensetzung, enthalten.

In einer weiteren bevorzugten Ausführungsform der Erfindung liegen die Monomeren und Oligomeren von Aminosäuren, N-C₂-C₂₄-Acylaminosäuren, den Estern und/oder den physiologisch verträglichen Metallsalzen dieser Substanzen, die nicht die Kollagensynthese stimulieren und nicht die Interaktion zwischen der extrazellulären Matrix und den Fibroblasten erhöhen und/oder verbessern, in geträgerter Form vor, insbesondere aufgetragen auf feinteiligen, pulverförmigen Substraten wie Kieselgel, insbesondere Aerosil-Typen, weiterhin Talkum, Microsponges, modifizierten Stärken und Stärkederivaten, kristalliner Cellulose, Cellulosepulvern, Lactoglobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen kosmetischen Wirkstoff enthalten, der ausgewählt ist aus DNA-Oligonucleotiden und RNA-Oligonucleotiden.
Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glycosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).
Ein erfindungsgemäß besonders bevorzugtes Oligonucleotid ist das Thymidin-Dinucleotid.
In den besonders bevorzugten erfindungsgemäßen Zusammensetzungen sind die DNA-Oligonucleotide oder RNA-Oligonucleotide in Mengen von 0,00001 - 5 Gew.-%, bevorzugt 0,0001 - 1,0 Gew.-% und besonders bevorzugt 0,0005 - 0,5 Gew.-%, bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen kosmetischen Wirkstoff enthalten, der ausgewählt ist aus mindestens einem Vitamin, Provitamin oder einer als Vitaminvorstufe bezeichnete Verbindung aus den Vitamingruppen B, E, H und K und den Estern der vorgenannten Substanzen.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden.

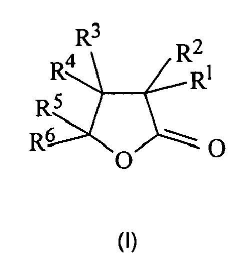

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄- Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (1) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.

Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.%, bezogen auf die gesamte Zusammensetzung, enthalten.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.
Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen kosmetischen Wirkstoff enthalten, der ausgewählt ist aus mindestens einer α-Hydroxycarbonsäure, α-Ketocarbonsäure oder β-Hydroxycarbonsäure oder deren Ester-, Lacton- und/ oder Salzform. Erfindungsgemäß geeignete α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl- , Dodecyl- und Hexadecylestern. Weitere Derivate der vorgenannten Säuren sind deren physiologisch verträglichen Salze, bevorzugt die Zink-, Kupfer- und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und Calcium-Salze.
Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.-%, besonders bevorzugt 0,5 - 5 Gew.-% und außerordentlich bevorzugt 1 - 2 Gew.-%, jeweils bezogen auf die gesamte Zusammensetzung, enthalten.
Die α-Hydroxycarbonsäuren, α-Ketocarbonsäuren oder β-Hydroxycarbonsäuren oder ihre Derivate zeigen im Zusammenwirken mit der erfindungsgemäßen Proteinhydrolysat-Dreierkombination a) bis c) eine überraschend bessere Verträglichkeit. Üblicherweise können die Säurekomponenten eine gewisse irritative Wirkung hervorrufen, insbesondere in höheren Konzentrationen. Diese kann durch die erfindungsgemäße Proteinhydrolysat-Dreierkombination a) bis c) verringert werden.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen kosmetischen Wirkstoff enthalten, der ausgewählt ist aus mindestens einem Flavonoid und/oder mindestens einem Flavonoid-reichen Pflanzenextrakt und der nicht die Kollagensynthese stimuliert und nicht die Interaktion zwischen der extrazellulären Matrix und den Fibroblasten erhöht und/oder verbessert. Die erfindungsgemäß bevorzugten Flavonoide umfassen die Glycoside der Flavone, der 3-Hydroxyflavone (Flavonole) und der Aurone. Die Flavanone und die Isoflavone sind erfindungsgemäß ausdrücklich von den Flavonoiden ausgenommen. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)flavon-3-(6-O-(6-deoxy-a-L-mannopyranosyl)-β-D-glucopyranosid)), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.
Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.
Die Flavonoide zeigen im Zusammenwirken mit der erfindungsgemäßen Proteinhydrolysat-Dreierkombination a) bis c) eine überraschend bessere antioxidative Wirkung, die die Antiaging-Leistung der Flavonoide heraufsetzt.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen kosmetischen Wirkstoff enthalten, der ausgewählt ist aus mindestens einem Ubichinon oder einem Ubichinol oder deren Derivaten. Ubichinole sind die reduzierte Form der Ubichinone. Die erfindungsgemäß bevorzugten Ubichinone weisen die Formel (11) auf: 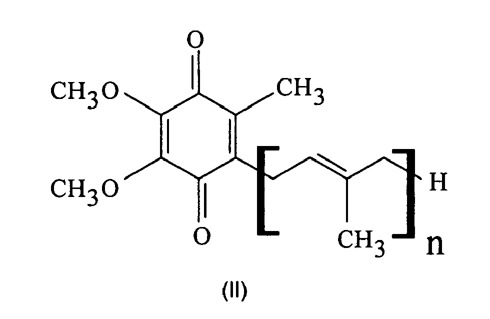 mit n = 6, 7, 8, 9 oder 10.

Besonders bevorzugt ist das Ubichinon der Formel (II) mit n = 10, auch bekannt als Coenzym Q10.
Erfindungsgemäß werden die Ubichinone, Ubichinole oder deren Derivate in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.
Die Ubichinone und Ubichinole zeigen im Zusammenwirken mit der erfindungsgemäßen Proteinhydrolysat-Dreierkombination a) bis c) eine überraschend bessere antioxidative Wirkung, die die Antiaging-Leistung der Flavonoide heraufsetzt.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin Silymarin enthalten. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Erfindungsgemäß wird Silymarin in Mengen von 0,0001 bis 1 Gew.-%, bevorzugt 0,001 bis 0,5 Gew.-% und besonders bevorzugt 0,005 bis 0,1 Gew.%, jeweils bezogen auf die gesamte Zusammensetzung, eingesetzt.
Silymarin zeigt im Zusammenwirken mit der erfindungsgemäßen Proteinhydrolysat-Dreierkombination a) bis c) eine überraschend bessere antioxidative Wirkung, die die Antiaging-Leistung des Silymarins heraufsetzt.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin Ectoin enthalten. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

Ectoin bewirkt im Zusammenwirken mit der erfindungsgemäßen Proteinhydrolysat-Dreierkombination a) bis c) eine überraschend bessere Nährstoffversorgung der Zellen der Haut.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens eine anorganische und/oder mindestens eine organische UV-Filtersubstanz enthalten.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.
Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/ Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethylhexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3', 5', 5', 5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.
Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.
Erfindungsgemäß sind die organischen UV-Filtersubstanzen bevorzugt in Mengen von 0,1 - 30 Gew.-%, besonders bevorzugt 0,5 - 20 Gew.-%, außerordentlich bevorzugt 1,0 - 15 Gew.-% und weiter bevorzugt 3,0 - 10 Gew.-%, jeweils bezogen auf die gesamte Zusammensetzung, enthalten.
Erfindungsgemäß sind die anorganischen UV-Filtersubstanzen bevorzugt in Mengen von 0,1-15 Gew.%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen selbstbräunenden Wirkstoff enthalten. Erfindungsgemäß bevorzugte selbstbräunende Wirkstoffe sind ausgewählt aus Dihydroxyaceton, Erythrulose und 5,6-Dihydroxyindolin.
Erfindungsgemäß sind die selbstbräunenden Wirkstoffe in einer Gesamtmenge von 0,01 - 15 Gew.-%, bevorzugt 0,1 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen hautberuhigenden Wirkstoff enthalten. Erfindungsgemäß bevorzugte hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI: Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter der Handelsbezeichnung Phytocohesine (INCI: Sodium Beta-Sitosterylsulfate) von der Firma Vincience erhältlich sind, Aminodicarbonsäuren mit einer C-Kettenlänge von 3-6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, sowie beliebigen Mischungen dieser Substanzen.
Die hautberuhigenden Wirkstoffe sind bevorzugt in Mengen von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen feuchtigkeitsspendenden Wirkstoff enthalten. Erfindungsgemäß bevorzugte feuchtigkeitsspendende Wirkstoffe sind ausgewählt aus Desoxyzuckern, besonders bevorzugt Rhamnose und Fucose, Polysacchariden, die mindestens einen Desoxyzucker-Baustein enthalten, besonders bevorzugt aus den Handelsprodukten Fucogel^{®} (INCI-Bezeichnung Biosaccharide Gum-1) von Solabia, Rhamnosoft^{®} (INCI-Bezeichnung Biosaccharide Gum-2) von Solabia, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, weiterhin Harnstoff, (2-Hydroxyethyl)harnstoff, Betain (Me₃N⁺-CH₂-COO⁻), Glycosaminoglycanen, besonders bevorzugt Hyaluronsäure, Dextran, Dextransulfat, Chondroitin-4-sulfat und Chondroitin-6-sulfat.

Die feuchtigkeitsspendenden Wirkstoffe sind bevorzugt in Gesamtmengen von 0,001 bis 10 Gew.-%, besonders bevorzugt 0,01 bis 5 Gew.-% und außerordentlich bevorzugt 0,1 bis 1 oder 2 Gew.-%, jeweils bezogen auf die gesamte Zusammensetzung, enthalten.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen sebumregulierenden Wirkstoff. Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques) und den Wirkstoffmischungen Asebiol^{®} BT 2 (Laboratoires Sérobiologiques, INCI: Aqua, Hydrolyzed Yeast Protein, Pyridoxine, Niacinamide, Glycerin, Panthenol, , Allantoin, Biotin) und Antifettfaktor^{®} COS-218/2-A (Cosmetochem, INCI: Aqua, Cetyl-PCA, PEG-8 Isolauryl Thioether, PCA, Cetyl Alcohol).
Die sebumregulierenden Wirkstoffe sind bevorzugt in einer Gesamtmenge von 0,001 bis 5 Gew.- %, besonders bevorzugt 0,01 bis 2 Gew.-% und außerordentlich bevorzugt 0,1 bis 1 Gew.-%, jeweils bezogen auf die gesamte Zusammensetzung, enthalten.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1 - 7 enthält, zur Behandlung lichtgeschädigter und/oder lichtgealterter und/oder intrinsisch und/oder extrinsisch gealterter Haut, insbesondere zur Antifaltenbehandlung.

Ein weiterer Gegenstand der vorliegenden Erfindung ist die Verwendung einer Kombination von a) mindestens einem Sojaproteinhydrolysat mit einem mittleren Molekulargewicht im Bereich von 1200 - 1800 Dalton, bevorzugt im Bereich von 1400 - 1700 Dalton, b) mindestens einem Sojaproteinhydrolysat mit einem mittleren Molekulargewicht im Bereich von 600 - 1000 Dalton, bevorzugt 800 Dalton, und c) mindestens einem Weizenproteinhydrolysat zur Herstellung einer kosmetischen oder dermatologischen Zusammensetzung zur Behandlung lichtgeschädigter und/oder lichtgealterter und/oder intrinsisch und/oder extrinsisch gealterter Haut, insbesondere zur Antifaltenbehandlung.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1-7 enthält, zur Steigerung der Hautfestigkeit und/oder zur Verbesserung der mechanischen Stabilität der Haut.

Ein weiterer Gegenstand der vorliegenden Erfindung ist die Verwendung einer Kombination von a) mindestens einem Sojaproteinhydrolysat mit einem mittleren Molekulargewicht im Bereich von 1200 - 1800 Dalton, bevorzugt im Bereich von 1400 - 1700 Dalton, b) mindestens einem Sojaproteinhydrolysat mit einem mittleren Molekulargewicht im Bereich von 600 - 1000 Dalton, bevorzugt 800 Dalton, und c) mindestens einem Weizenproteinhydrolysat zur Herstellung einer kosmetischen oder dermatologischen Zusammensetzung zur Steigerung der Hautfestigkeit und/oder zur Verbesserung der mechanischen Stabilität der Haut.

Die Hautfestigkeit und die mechanische Stabilität der Haut kann zum einen z. B. über den Torsionswinkel, der mit dem Dermal Torque Meter gemessen werden kann, zum anderen über den Parameter U_{F} (totale Ausdehnbarkeit der Haut bei Unterdruck), der mit dem Cutometer gemessen werden kann, bestimmt werden.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1 - 7 enthält, zur Steigerung der Hautelastizität und/oder der Hautstraffheit und/oder zur Verbesserung der Hautweichheit und Hautgeschmeidigkeit, jeweils bezogen auf die oberen Hautschichten.

Ein weiterer Gegenstand der vorliegenden Erfindung ist die Verwendung einer Kombination von a) mindestens einem Sojaproteinhydrolysat mit einem mittleren Molekulargewicht im Bereich von 1200 - 1800 Dalton, bevorzugt im Bereich von 1400 - 1700 Dalton, b) mindestens einem Sojaproteinhydrolysat mit einem mittleren Molekulargewicht im Bereich von 600 - 1000 Dalton, bevorzugt 800 Dalton, und c) mindestens einem Weizenproteinhydrolysat zur Herstellung einer kosmetischen oder dermatologischen Zusammensetzung zur Steigerung der Hautelastizität und/oder der Hautstraffheit und/oder zur Verbesserung der Hautweichheit und Hautgeschmeidigkeit, jeweils bezogen auf die oberen Hautschichten, und/oder zur Glättung der Haut und/oder zur Verminderung von Falten, Fältchen und/oder feinen Linien und/oder zum Schutz und/oder zur Prophylaxe vor extrinsischer Hautalterung und/oder zur Verzögerung der extrinsischen Hautalterung und/oder zur Verzögerung der intrinsischen Hautalterung und/oder zur Behandlung von Altersflecken und/oder zur Aufrechterhaltung des normalen Proteinstoffwechsels der Haut und/oder zur Verminderung oxidativer Schäden in der Haut.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600- 1000 Dalton, bevorzugt 800 Dalton, und c) mindestens ein Weizenproteinhydrolysat gemäß einem der Patentansprüche 1 - 7 enthält, zur Glättung der Haut und/oder zur Verbesserung des optischen Erscheinungsbildes der Haut

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1 - 7 enthält, zur Verminderung von Falten, Fältchen und/oder feinen Linien.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1 - 7 enthält, zur Verbesserung des optischen Erscheinungsbildes der Haut.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1 - 7 enthält, zur Verbesserung der Hautverträglichkeit von Hautbehandlungsmitteln mit Antiageing-Wirkung, insbesondere von Antifaltenmitteln.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1 - 7 enthält, zum Schutz und/oder zur Prophylaxe vor extrinsischer Hautalterung und/oder zur Verzögerung der extrinsischen Hautalterung.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1 - 7 enthält, zur Verzögerung der intrinsischen Hautalterung.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1 - 7 enthält, zur Behandlung von Altersflecken.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1 - 7 enthält, zur Aufrechterhaltung des normalen Proteinstoffwechsels der Haut.

Ein weiterer Gegenstand der vorliegenden Erfindung ist die nicht-therapeutische, kosmetische Verwendung einer kosmetischen oder dermatologischen Zusammensetzung zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat gemäß einem der Patentansprüche 1 - 7 enthält, zur Verminderung oxidativer Schäden in der Haut.

Erfindungsgemäß besonders bevorzugte kosmetische oder dermatologische Zusammensetzungen zur topischen Behandlung der Haut, die in einem geeigneten kosmetischen oder dermatologischen Träger a) mindestens ein Sojaproteinhydrolysat mit einem mittleren Molekulargewicht im Bereich von 1200 - 1800 Dalton, bevorzugt im Bereich von 1400 - 1700 Dalton, b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat enthalten, sind dadurch gekennzeichnet, dass sie weiterhin mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.
Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4-30 Kohlenstoffatomen, die mit 1 - 75, bevorzugt 5-20 Ethylenoxid-Einheiten ethoxyliert und/oder mit 3 - 30, bevorzugt 9-14 Propylenoxid-Einheiten propoxyliert sein können, Esterölen, das heißt Estern von C₆₋₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1-10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, EiLecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.
Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf das gesamte Hautbehandlungsmittel.

Bevorzugt liegen die erfindungsgemäßen kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.
In einer besonders bevorzugten Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-ÖI-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.
In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI), polymere Emulgatoren wie PEG-150-distearat oder PEG-30-dipolyhydroxystearat, sowie lineare und verzweigte C₈-C₃₀-Fettsäuren und deren Na-, K-, Ammonium-, Ca-, Mg- und Zn - Salze.

Die erfindungsgemäßen Zusammensetzungen enthalten mindestens einen Emulgator bevorzugt in einer Gesamtmenge von 0,1 bis 25 Gew.-%, besonders bevorzugt 0,5 - 15 Gew.-%, bezogen auf die gesamte Zusammensetzung.
In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹- O - R², in der R¹ eine primäre lineare Alkyl-, Alkenyl- oder Acylgruppe mit 20 - 30C-Atomen, bevorzugt ein Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6C-Atomen und 2 - 5 Hydroxylgruppen, bevorzugt Wasserstoff, darstellt. Eine besonders bevorzugte Verbindung R¹- O - R² ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythritmonoerucat. Die Anwesenheit von Emulgatoren der allgemeinen Formel R¹- O - R² kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher dadurch gekennzeichnet, dass sie in Form einer lamellare Strukturen aufweisenden Öl-in-Wasser-Emulsion vorliegen.
Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342, 1382 und ETD 2020 (ex B.F. Goodrich).
Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden. Weitere bevorzugte Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

### Experimentelle Bestimmung der Interaktion zwischen der extrazellulären Matrix und den Fibroblasten mit Hilfe eines Kollagengels

Die Kombination von nativem Kollagen Typ I und humanen Vorhaut-Fibroblasten bildet ein dreidimensionales, kontraktionsfähiges Gelsystem. Die Fibroblasten-vermittelte Gelkontraktion wird durch eine zeitabhängige Veränderung der Gelfläche beobachtet. Das heißt, eine stärkere Kontraktion des Kollagen-Gels deutet auf eine stärkere Interaktion zwischen den Fibroblasten und der Kollagenmatrix hin. Übertragen auf eine in vivo-Kollagenmatrix, bedeutete das, dass das Kollagen unter stärkerer mechanischer Spannung stehen und besser die Haut straffen würde.

### Versuchsdurchführung

Normale Vorhaut-Fibroblasten wurden als Monolayer mit DMEM (Dulbecco's Modified Eagle's Medium) /10 Gew.-% FCS (fötalem Kälberserum) kultiviert. 24 Stunden vor Einsaat in das Kollagengel wurden die Zellen in Medium kultiviert, dem die entsprechenden Prüfsubstanzen zugesetzt wurden. Vor Zugabe in den Kollagengelansatz wurden die Zellen trypsiniert, die entsprechende Zellzahl wurde in 50ml Faicon-Röhrchen zweimal mit Phosphat-gepufferter Kochsalzlösung gewaschen, abschließend zentrifugiert und in DMEM (Leermedium) resuspendiert. Kollagen Typ I aus Kälberhaut (IBFB, Leipzig) wurde in 0,1 M Essigsäure gelöst, so dass eine Konzentration von 3 mg/ml vorlag.
Zur Bestimmung der Gelkontraktion wurde die Kollagen-Zellmischung luftblasenfrei in unbeschichtete Schalen gegeben. Der Durchmesser der Kollagengele wurde zu verschiedenen Zeitpunkten bestimmt (0, 1, 2, 3, 4, 5, 6 und 24 Stunden). Aus den mittleren Durchmessern wurde die Fläche der Gele berechnet, als Grundlage für weitere Auswertungen. Für jede Prüfsubstanz wurden jeweils 3 Kollagengele mit einer Konzentration an fötalem Kälberserum von 0,5 Gew.-% angesetzt. Die in Tabelle 1 aufgeführten Mengenangaben beziehen sich auf das eingesetzte Handelsprodukt als solchem.

Die Kurvenverläufe wurden mittels K50-Werte und Berechnung der Area under Curve (AUC) systematisch analysiert. Als Bezugspunkt wurde jeweils die Kontrolle mit 0,5 Gew.-% FCS herangezogen. Der K50-Wert beschreibt, zu welchem Zeitpunkt die Gelfläche die Hälfte (50%) ihrer Ausgangsfläche (100% = 22 cm²) erreicht hat, also einen flexiblen Punkt in den unterschiedlichen Kontraktionskurven. Die Hälfte der Fläche wurde bei allen Gelen innerhalb der ersten 5 Stunden erreicht. Der K50-Wert der Kontrolle mit 0,5 Gew.-% fötalem Kälberserum wird für alle Kontraktionsversuche als Vergleichswert (= 100%) angesehen. Die Daten entsprechen den Mittelwerten (n = 3); "n.b." bedeutet "nicht bestimmt".

**Tabelle 1: Zeitlicher und qualitativer Verlauf der Kontraktion des Kollagengels unter dem Einfluss verschiedener Wirkstoffe, die die Interaktion zwischen der extrazellulären Matrix und den Fibroblasten erhöhen und/oder verbessern**

| | K50 [%] | AUC [%] |
|---|---|---|
| Kontrolle | 100 | 100 |
| Carnitin (100 µg/ml) | 86 | 69 |
| Retinsäure (1 µg/ml) | 89 | 74 |
| Vitamin C (50 µg/ml) | 83 | 66 |
| Keratec Pep (Wollkeratinhydrolysat) (5 mg/ml) | 87 | 76 |
| Pearl Protein Extract (Conchiolin-Hydrolysat) (250 µg/ml) | 83 | 65 |
| Lipobelle Soyaglycone (1 mg/ml) | 88 | 83 |
| Crodarom Chardonnay (50 µg/ml) | 93 | n.b. |
| Ridulisse C (Sojaprotein-Hydrolysat mit einer mittleren Molmasse von 1200 - 1700 Dalton) (20 mg/ml) | n.b. | 89 |
| Sambucus AO (Schwarzer Holunderblüten-Extrakt) (10 mg/ml) | n.b. | 85 |
| Coccopolipeptide di Soja (Potassium Cocoyl Hydrolyzed Soy Protein) (20 µg/ml) | n.b. | 85 |
| Caomint (5 mg/ml) | n.b. | 88 |
| Lotus Germ Extract BG (Lotuskeim-Extrakt) (250 µg/ml) | n.b. | 92 |

Die Untersuchungen zur Verbesserung der Hautweichheit und Hautgeschmeidigkeit, also der "Elastizität", der oberen Hautschicht wurden an 40 Probandinnen nach einmaliger Applikation der erfindungsgemäßen Testcreme am Unteram mittels Hauttorsionsmessungen über eine Dauer von 10 Stunden durchgeführt.

| Zusammensetzung der erfindungsgemäßen Testcreme | |
|---|---|
| Montanov 68 | 5,00 |
| Caprylic/Capric Triglycerides | 5,00 |
| Cetearyl Alcohol | 1,00 |
| Cutina MD | 2,00 |
| Baysilone-Öl M 350 | 1,00 |
| Tocopherylacetat | 0,50 |
| Shea butter | 0,50 |
| Novata AB | 2,00 |
| Controx KS | 0,25 |
| Methylbenzylidene Camphor | 2,00 |
| Butylmethoxydibenzoylmethan | 1,00 |
| Propylparaben | 0,20 |
| Glycerin 86 % | 4,50 |
| 1,6-Hexandiol | 6,00 |
| Methylparaben | 0,20 |
| Talkum | 0,50 |
| Tego Carbomer 140 | 0,50 |
| Phenoxyethanol | 0,40 |
| Parfum | 0,40 |
| Probiol L 01 | 1,00 |
| DSH-C N | 2,00 |
| Trinatriumnitriloacetat (40 %) | 0,10 |
| Gatuline RC | 2,00 |
| Phytokine | 1,50 |
| Ridulisse C | 0,50 |
| Liftiline | 3,00 |
| Dry Flo Plus | 1,00 |
| Simulgel NS | 1,00 |
| Natriumhydroxid | 0,045 |
| Wasser | ad 100 |

Die erfindungsgemäße Testcreme wurde in standardisierter Menge einmalig auf die definierten Areale des Unterarms aufgetragen (0,1 ml Testcreme pro 25 cm²) und die Hautareale vor der Applikation sowie 6, 8 und 10 Stunden nach der Applikation vermessen. Die Applikation erfolgte randomisiert auf dem rechten bzw. linken Arm. Der Wirkstoff-haltigen Formulierung wurde ein korrespondierendes unbehandeltes Areal zugeordnet, so dass bei der statistischen Analyse ein Many-to-one-Vergleich der Ausgangslagendifferenzen ausgeführt werden konnte.
Als Indikator für die Hautgeschmeidigkeit diente der Quotient der beiden Messparameter Uᵣ und Uₑ. Die Verbesserung der Hautgeschmeidigkeit ist an der Zunahme des Quotienten Uᵣ / Uₑ erkennbar.

Die Messergebnisse der Hauttorsionsmessungen sind in der folgenden Tabelle zusammengestellt.

| Messzeitpunkt | Uᵣ / Uₑ | Uᵣ / Uₑ |
|---|---|---|
| | behandelt | unbehandelt |
| vor Applikation | 0,40 | 0,38 |
| 6 Stunden nach Applikation | 0,45 | 0,38 |
| 8 Stunden nach Applikation | 0,44 | 0,39 |
| 10 Stunden nach Applikation | 0,43 | 0,40 |

Die statistische Analyse der Ausgangslagendifferenzen von behandeltem und unbehandeltem Areal zeigte signifikante positive Effekte 6 Stunden und 8 Stunden nach der Applikation (p-Werte 0,000016 bzw. 0,003725), das heißt, die erfindungsgemäße Testcreme führte nach einmaliger Applikation über einen Zeitraum von 8 Stunden zu einer signifikanten Verbesserung der Geschmeidigkeit und Hautstraffheit in den oberen Hautschichten.

Die nachfolgenden Formulierungsbeispiele sollen den Gegenstand der Erfindung erläutern, ohne ihn hierauf zu beschränken.

Alle Mengenangaben sind in Gew.-%, bezogen auf die gesamte Zusammensetzung.

### 1. Oel-in-Wasser-Emulsionen

### 1. Hautcremes

| | 1 | 2 | 3 | 4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Baysilone-Öl M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Algenextrakt | 1,00 | - | - | - |
| Caomint | 1,00 | 0,50 | 2,00 | - |
| Calmosensine | 1,00 | 2,00 | - | 2,00 |
| Symdiol 68 | 0,30 | 0,50 | - | - |
| DSH-CN | 5,00 | 5,00 | 5,00 | 5,00 |
| Hydrovance | 4,30 | 2,50 | 8,60 | 10,00 |
| Kombuchka | 3,00 | - | - | 3,00 |
| Glistin | - | 2,00 | - | - |
| Natrulon RC 50 DG | - | - | 1,00 | - |
| Phytokine | 2,00 | 1,50 | 2,00 | 2,00 |
| Ridulisse C | 0,50 | 0,50 | 0,50 | 0,50 |
| Liftiline | 2,00 | 3,00 | 2,00 | 3,00 |
| Matrixyl 3000 | - | 2,00 | - | - |
| Olea europ. Fol extr. S. sicc. | - | - | - | 0,10 |
| Perfume | 0,10 | 0,10 | 0,10 | 0,10 |
| Aqua | ad 100 | ad 100 | ad 100 | ad 100 |

### 2. Hautcremes:

| | 1 | 2 | 3 | 4 | 5 | 6 |
|---|---|---|---|---|---|---|
| Cetearyl Isononanoate | 4,00 | 4,00 | 4,00 | 4,00 | 4,00 | 4,00 |
| Mineral oil | 6,00 | 6,00 | 6,00 | 6,00 | 6,00 | 6,00 |
| Cutina CBS | 2,00 | 2,00 | 2,00 | 2,00 | 2,00 | 2,00 |
| Palmitic Acid / Stearic Acid | 1,50 | 1,50 | 1,50 | 1,50 | 1,50 | 1,50 |
| Eumulgin B3 | 1,00 | 1,00 | 1,00 | 1,00 | 1,00 | 1,00 |
| Baysilone-Öl M 350 | 1,00 | 1,00 | 1,00 | 1,00 | 1,00 | 1,00 |
| Tocopheryl Acetate | 0,50 | 0,50 | 0,50 | 0,50 | 0,50 | 0,50 |
| Propylparaben | 0,30 | 0,30 | 0,30 | 0,30 | 0,30 | 0,30 |
| Pemulen TR 1 | 0,27 | 0,27 | 0,27 | 0,27 | 0,27 | 0,27 |
| Glycerin | 5,00 | 3,00 | 3,00 | 5,00 | 3,00 | 3,00 |
| Milchsäure 80 % | 0,26 | 0,26 | 0,26 | 0,26 | 0,26 | 0,26 |
| Propylenglycol | 5,00 | 5,00 | 5,00 | 5,00 | 5,00 | 5,00 |
| Methylparaben | 0,30 | 0,30 | 0,30 | 0,30 | 0,30 | 0,30 |
| Phenoxyethanol | 0,90 | 0,90 | 0,90 | 0,90 | 0,90 | 0,90 |
| Phytokine | 2,00 | 1,50 | 2,00 | 2,00 | 1,50 | 2,00 |
| Ridulisse C | 0,50 | 0,50 | 0,50 | 0,50 | 0,50 | 0,50 |
| Liftiline | 2,00 | 3,00 | 2,00 | 2,00 | 3,00 | 2,00 |
| Sepigel 305 | 0,50 | 0,50 | 0,50 | 0,50 | 0,50 | 0,50 |
| Silk Protein | 0,25 | 0,25 | 0,25 | 0,25 | 0,25 | 0,25 |
| Taurin | 1,00 | 0,50 | 2,00 | 1,00 | 0,50 | 2,00 |
| Calmosensine | 1,00 | 2,00 | - | 1,00 | 2,00 | - |
| Keltrol SF | 0,20 | 0,20 | 0,20 | 0,20 | 0,20 | 0,20 |
| (2-Hydroxyethyl)harnstoff | 4,30 | 2,50 | 8,60 | - | - | - |
| Hydrovance | - | - | - | 4,30 | 2,50 | 8,60 |
| Perfume | 0,30 | 0,30 | 0,30 | 0,30 | 0,30 | 0,30 |
| Natrium Ascorbylphosphat | 1,00 | - | 1,00 | 1,00 | - | 1,00 |
| Keratec Pep | - | 2,00 | - | - | 2,00 | - |
| Kreatin | - | - | 1,00 | - | - | 1,00 |
| Retinol | 0,10 | - | - | 0,10 | - | - |
| Deliner | - | 2,00 | - | - | 2,00 | - |
| Aqua | ad 100 | ad 100 | ad 100 | ad 100 | ad 100 | ad 100 |

### 3. Tagescremes:

| | 1 | 2 | 3 | 4 | 5 |
|---|---|---|---|---|---|
| Lipoid S 75-3 | 0,50 | 0,50 | 0,50 | 0,50 | 0,50 |
| Isopropylstearate | 4,00 | 4,00 | 4,00 | 4,00 | 4,00 |
| Dibutyl Adipate | 2,00 | 2,00 | 2,00 | 2,00 | 2,00 |
| Tocopheryl Acetate | 0,50 | 0,50 | 0,50 | 0,50 | 0,50 |
| Cutina MD | 1,00 | 1,00 | 1,00 | 1,00 | 1,00 |
| Behenyl Alcohol | 2,00 | 2,00 | 2,00 | 2,00 | 2,00 |
| Baysilone-ÖI M 350 | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerol | 3,00 | 4,50 | 3,00 | 3,00 | 3,00 |
| Hexandiol | 6,00 | 3,00 | - | - | 4,00 |
| Methylparaben | 0,20 | 0,20 | 0,20 | 0,20 | 0,20 |
| Tego Carbomer | 0,30 | 0,30 | 0,30 | 0,30 | 0,30 |
| Algae Extract | 1,00 | - | - | - | - |
| Photosomes | 0,20 | 0,20 | 0,20 | 0,20 | 0,20 |
| Dimethylmethoxy Chromanol | 0,01 | 0,01 | 0,01 | 0,01 | 0,01 |
| Propylenglycol | 5,00 | 5,00 | 5,00 | 5,00 | 5,00 |
| Phytokine | 2,00 | 1,50 | 2,00 | 2,00 | 1,50 |
| Ridulisse C | 0,50 | 0,50 | 0,50 | 0,50 | 0,50 |
| Liftiline | 2,00 | 3,00 | 2,00 | 2,00 | 3,00 |
| Caomint | 1,00 | - | 2,00 | 2,00 | 2,00 |
| Calmosensine | 1,00 | 2,00 | - | 1,00 | 1,00 |
| Kombuchka | 3,00 | 3,00 | - | - | 2,00 |
| Natriumascorbylphosphat | - | - | 1,00 | 1,00 | 1,00 |
| Glistin | - | 2,00 | - | - | - |
| Natrulon RC50DG | - | - | - | - | 1,00 |
| Deliner | - | - | 2,00 | 1,00 | - |
| Matrixyl 3000 | 1,00 | 1,00 | - | - | 1,00 |
| Simulgel NS | 1,50 | 1,50 | 1,50 | 1,50 | 1,50 |
| Sodium Benzoate | - | 0,20 | 0,50 | - | - |
| Phenoxyethanol | 0,50 | - | - | - | - |
| Symdiol 68 | 0,30 | 0,50 | 0,30 | - | - |
| TiO₂ | 0,50 | 0,50 | - | - | - |
| Hydrovance | 2,50 | 4,30 | 8,60 | 8,60 | 8,60 |
| Silymarin Phytosome | 0,30 | - | - | - | 0,50 |
| Ectoin | 0,50 | - | 0,50 | 0,50 | 0,50 |
| Vitamin B6 | - | - | 0,01 | 0,01 | 0,01 |
| Perfume | 0,35 | 0,35 | 0,35 | 0,35 | 0,35 |
| Aqua | ad 100 | ad 100 | ad 100 | ad 100 | ad 100 |

### 4. Tagescremes mit Lichtschutzfaktor:

| | 1 | 2 | 3 | 4 | 5 | 6 | 7 | 8 | 9 | 10 | 11 |
|---|---|---|---|---|---|---|---|---|---|---|---|
| Montanov 68 | 5,00 | 5,00 | 5,00 | 5,00 | 5,00 | 5,00 | 5,00 | 5,00 | 5,00 | 5,00 | 5,00 |
| Myritol 318 | 5,00 | 5,00 | 5,00 | 5,00 | 5,00 | 5,00 | 5,00 | 5,00 | 5,00 | 5,00 | 5,00 |
| Shea Butter | 0,50 | 0,50 | 0,50 | 0,50 | 0,50 | 0,50 | 0,50 | 0,50 | 0,50 | 0,50 | 0,50 |
| Novata AB | 2,00 | 2,00 | 2,00 | 2,00 | 2,00 | 2,00 | 2,00 | 2,00 | 2,00 | 2,00 | 2,00 |
| Cetearyl Alkohol | 1,00 | 1,00 | 1,00 | 1,00 | 1,00 | 1,00 | 1,00 | 1,00 | 1,00 | 1,00 | 1,00 |
| Cutina MD | 2,00 | 2,00 | 2,00 | 2,00 | 2,00 | 2,00 | 2,00 | 2,00 | 2,00 | 2,00 | 2,00 |
| Dimethicone | 0,50 | 0,50 | 0,50 | 0,50 | 0,50 | 0,50 | 0,50 | 0,50 | 0,50 | 0,50 | 0,50 |
| Tocopherylacetat | 0,50 | 0,50 | 0,50 | 0,50 | 0,50 | 0,50 | 0,50 | 0,50 | 0,50 | 0,50 | 0,50 |
| Controx KS | 0,25 | 0,25 | 0,25 | 0,25 | 0,25 | 0,25 | 0,25 | 0,25 | 0,25 | 0,25 | 0,25 |
| Caomint | 1,00- | - | 2,00 | 2,00 | 1,00 | - | 2,00 | 2,00 | 1,00 | - | 2,00 |
| Calmosensine | 1,00 | 2,00 | - | 1,00 | 1,00 | 2,00 | - | 1,00 | 1,00 | 2,00 | - |
| Phytokine | 2,00 | 1,50 | 2,00 | 2,00 | 1,50 | 2,00 | 1,50 | 2,00 | 2,00 | 1,50 | 1,50 |
| Ridulisse C | 0,50 | 0,50 | 0,50 | 0,50 | 0,50 | 0,50 | 0,50 | 0,50 | 0,50 | 0,50 | 0,50 |
| Liftiline | 2,00 | 3,00 | 2,00 | 2,00 | 3,00 | 2,00 | 3,00 | 2,00 | 2,00 | 3,00 | 3,00 |
| Polysilicone-15 | - | 4,00 | 4,00 | - | - | 4,00 | 4,00 | - | - | 4,00 | - |
| Phenylbenzimidazole Sulfonic Acid | - | 2,00 | 2,00 | 2,00 | - | 2,00 | - | 2,00 | 2,00 | - | - |
| Disodium Phenyl Dibenzimidazole Tetrasulfonate | - | - | - | 1,00 | - | - | - | - | - | - | - |
| Octocrylene | - | - | - | 5,00 | - | 5,00 | - | - | - | - | 5,00 |
| Octyl Triazone | - | - | - | - | - | - | - | 5,00 | - | - | 2,00 |
| Octylsalicylate | | | | | | | | | | 2,00 | - |
| Diethylamino Hydroxybenzoyl Hexyl Benzoat | - | - | - | - | - | - | - | - | - | - | 2,50 |
| 4-Methoxy benzylidene Camphor | 2,00 | - | - | - | - | - | - | - | - | - | - |
| Butyl Methoxy- Dibenzoyl-methane | 1,00 | 1,80 | 1,80 | - | - | - | 1,80 | 1,80 | 1,80 | 1,80 | - |
| TiO₂ | - | - | - | - | - | - | 2,00 | - | 2,00 | - | - |
| Propylparaben | 0,20 | 0,20 | 0,20 | 0,20 | 0,20 | 0,20 | 0,20 | 0,20 | 0,20 | 0,20 | 0,20 |
| Tego Carbomer | 0,50 | 0,50 | 0,50 | 0,50 | 0,50 | 0,50 | 0,50 | 0,50 | 0,50 | 0,50 | 0,50 |
| Hexandiol | 6,00 | 6,00 | 3,00 | 6,00 | | 6,00 | 6,00 | 6,00 | 6,00 | 6,00 | 6,00 |
| Talc | 0,50 | 0,50 | 0,50 | 0,50 | 0,50 | 0,50 | 0,50 | 0,50 | 0,50 | 0,50 | 0,50 |
| Methylparaben | 0,20 | 0,20 | 0,20 | 0,20 | 0,20 | 0,20 | 0,20 | 0,20 | 0,20 | 0,20 | 0,20 |
| Glycerol | 4,50 | 4,50 | 3,00 | 4,50 | 3,00 | 4,50 | 4,50 | 4,50 | 4,50 | 4,50 | 4,50 |
| Dry Flo Plus | 1,00 | 1,00 | 1,00 | 1,00 | 1,00 | 1,00 | 1,00 | 1,00 | 1,00 | 1,00 | 1,00 |
| Hydrogenated Lecithin | 1,00 | 1,00 | 1,00 | 1,00 | 1,00 | 1,00 | 1,00 | 1,00 | 1,00 | 1,00 | 1,00 |
| Hydrolyzed Protein | 5,00 | 5,00 | 5,00 | 5,00 | 5,00 | 5,00 | 5,00 | 5,00 | 5,00 | 5,00 | 5,00 |
| Trisodium NTA | 0,10 | 0,10 | 0,10 | 0,10 | 0,10 | 0,10 | 0,10 | 0,10 | 0,10 | 0,10 | 0,10 |
| Phenoxyethanol | 0,40 | 0,40 | 0,40 | 0,40 | 0,40 | 0,40 | 0,40 | 0,40 | 0,40 | 0,40 | 0,40 |
| Hydrovance | 4,30 | 4,30 | 2,50 | 8,60 | 8,60 | 4,30 | 4,30 | 2,50 | 8,60 | 8,60 | 4,30 |
| Symdiol 68 | 0,30 | 0,30 | 0,30 | 0,50 | - | - | - | 0,50 | - | - | 0,50 |
| Kombuchka | 3,00 | 3,00 | - | 3,00 | 3,00 | 2,00 | - | - | 2,00 | 3,00 | |
| Glistin | 2,00 | - | 2,00 | 2,00 | - | 1,00 | - | - | - | - | 2,00 |
| Keratec Pep | - | - | - | - | - | - | 2,00 | 2,00 | 1,00 | - | - |
| Kreatin | - | 1,00 | - | - | - | - | 1,00 | - | - | 1,00 | - |
| Retinol | 0,10 | - | - | - | - | - | - | - | 0,10 | - | - |
| Olea europ. Fol extr. S. sicc. | 0,10 | - | - | - | - | - | - | 0,10 | - | - | 0,10 |
| SYN-COLL | - | - | 1,00 | 1,00 - | - | - | - | - | - | - | - |
| Matrixyl 3000 | - | - | - | - | 1,00 | 2,00 | - | - | 1,00 | 2,00 | - |
| Vegetal Filling Spheres | - | - | - | 1,00 | 1,00 | 1,00 | 1,00 | - | - | - | - |
| Biotin | 0,1 | - | - | 0,1 | - | - | - | - | - | - | - |
| Perfume | 0,40 | 0,40 | 0,40 | 0,40 | 0,40 | 0,40 | 0,40 | 0,40 | 0,40 | 0,40 | 0,40 |
| Aqua | ad 100 | ad 100 | ad 100 | ad 100 | ad 100 | ad 100 | ad 100 | ad 100 | ad 100 | ad 100 | ad 100 |

### 5. Tagescremes:

| | 1 | 2 | 3 | 4 | 5 | 6 |
|---|---|---|---|---|---|---|
| Montanov L | 3,00 | 3,00 | 3,00 | 3,00 | 3,00 | 3,00 |
| Dibutyl Adipate | 6,00 | 6,00 | 6,00 | 6,00 | 6,00 | 6,00 |
| Myritol 318 | 3,00 | 3,00 | 3,00 | 3,00 | 3,00 | 3,00 |
| Cetearyl Alkohol | 1,00 | 1,00 | 1,00 | 1,00 | 1,00 | 1,00 |
| Glyceryl Stearate (Cutina MD) | 0,50 | 0,50 | 0,50 | 0,50 | 0,50 | 0,50 |
| Dimethicone (Baysilone M350) | 0,50 | 0,50 | 0,50 | 0,50 | 0,50 | 0,50 |
| Propylparaben | 0,20 | 0,20 | 0,20 | 0,20 | 0,20 | 0,20 |
| Cyclopentasiloxane | 1,50 | 1,50 | 1,50 | 1,50 | 1,50 | 1,50 |
| Glycerol | 5,00 | 5,00 | 5,00 | 5,00 | 5,00 | 5,00 |
| Sorbitol | 3,00 | 3,00 | 3,00 | 3,00 | 3,00 | 3,00 |
| Methylparaben | 0,20 | 0,20 | 0,20 | 0,20 | 0,20 | 0,20 |
| Dry Flo Plus | 0,50 | 0,50 | 0,50 | 0,50 | 0,50 | 0,50 |
| Sodium Carbomer | 0,10 | 0,10 | 0,10 | 0,10 | 0,10 | 0,10 |
| Caomint | 1,00 | - | 2,00 | 1,00 | - | 2,00 |
| Calmosensine | 1,00 | 2,00 | - | 1,00 | 2,00 | - |
| Laminata Digitata Extract | 0,50 | - | - | - | - | - |
| Hydrovance | 2,50 | 4,30 | 8,60 | 2,50 | 4,30 | - |
| Matrixyl 3000 | 1,00 | 1,00 | 0,30 | 1,00 | 1,00 | - |
| Photosomes | 3,00 | - | - | 3,00 | - | - |
| Vegetal Filling Spheres | 1,00 | 2,00 | - | 1,00 | 2,00 | - |
| Betain | - | - | 0,50 | - | - | - |
| Perfume | 0,20 | 0,20 | 0,20 | 0,20 | 0,20 | - |
| Retinylpalmitat | 0,10 | 0,10 | 0,10 | 0,10 | 0,10 | - |
| Pantolacton | - | 0,50 | - | - | 0,50 | - |
| Ascorbylpalmitat | - | - | - | - | - | 1,00 |
| Bisabolol | 0,50 | 0,50 | 0,50 | 0,50 | - | - |
| Pearl Protein Extract | 1,00 | - | - | 2,00 | 1,00 | - |
| Ridulisse C | 0,50 | 1,00 | 1,00 | 0,50 | 1,00 | 0,50 |
| Phytokine | 1,50 | 2,00 | 1,50 | 1,50 | 1,50 | 1,50 |
| Liftiline | 3,00 | 3,00 | 3,00 | 2,50 | 2,50 | 2,50 |
| Exsy Algine | - | - | 2,00 | 1,00 | - | - |
| Sepilift DPHP | - | - | - | - | - | 1,00 |
| Matrixyl 3000 | 1,00 | - | - | 1,00 | - | 1,00 |
| Retinol | - | - | 0,20 | - | 0,10 | - |
| Aqua | ad 100 | ad 100 | ad 100 | ad 100 | ad 100 | ad 100 |

### Hautcremes auf Basis einer Lipoprotein-Creme

| | 1 | 2 | 3 | 4 | 5 | 6 |
|---|---|---|---|---|---|---|
| Myritol^{®} PC | 3,5 | 3,5 | 3,5 | 3,5 | 3,5 | 3,5 |
| Lanette^{®} 22 | 3,0 | 3,0 | 3,0 | 3,0 | 3,0 | 3,0 |
| Cutina^{®} GMS-V | 3,0 | 3,0 | 3,0 | 3,0 | 3,0 | 3,0 |
| Stenol^{®} 16/18 | 2,0 | 2,0 | 2,0 | 2,0 | 2,0 | 2,0 |
| Distelöl | 3,0 | 3,0 | 3,0 | 3,0 | 3,0 | 3,0 |
| Controx^{®} KS | 0,05 | 0,05 | 0,05 | 0,05 | 0,05 | 0,05 |
| Baysilon^{®} M350 | 1,0 | 1,0 | 1,0 | 1,0 | 1,0 | 1,0 |
| Isopropylstearat | 6,0 | 6,0 | 6,0 | 6,0 | 6,0 | 6,0 |
| Propylparaben | 0,2 | 0,2 | 0,2 | 0,2 | 0,2 | 0,2 |
| Caomint | 1,00 | - | 2,00 | 1,00 | - | 2,00 |
| Calmosensine | 1,00 | 2,00 | - | 1,00 | 2,00 | - |
| Ridulisse C | 0,50 | 1,00 | 1,00 | 0,50 | 1,00 | 0,50 |
| Phytokine | 1,50 | 2,00 | 1,50 | 1,50 | 1,50 | 1,50 |
| Liftiline | 3,00 | 3,00 | 3,00 | 2,50 | 2,50 | 2,50 |
| Glycerin | 5,0 | 5,0 | 5,0 | 5,0 | 5,0 | 5,0 |
| Ethoxydiglycol | 5,0 | 5,0 | 5,0 | 5,0 | 5,0 | 5,0 |
| Methylparaben | 0,2 | 0,2 | 0,2 | 0,2 | 0,2 | 0,2 |
| Hibiscin^{®} HP LS 9198 | 3,0 | 3,0 | 3,0 | 3,0 | 3,0 | 3,0 |
| Citronensäure | 0,1 | 0,1 | 0,1 | 0,1 | 0,1 | 0,1 |
| Dihydroxyaceton | 0,5 | - | - | - | - | - |
| Fucogel 1000 | 2,0 | 1,0 | 1,0 | 1,0 | - | 1,0 |
| Sepigel^{®} 305 | 2,0 | 2,0 | 2,0 | 2,0 | 2,0 | 2,0 |
| Pearl Protein Extract | - | 2,00 | - | - | 1,00 | - |
| Glistin | 1,00 | - | 2,00 | 2,00 | - | - |
| Kombuchka | 3,00 | - | - | 3,00 | 3,00 | - |
| Natriumascorbylphosphat | - | - | - | - | - | 1,00 |
| Retinol | - | 0,25 | | 0,10 | | 0,15 |
| Deliner | 1,00 | - | 1,00 | - | 1,00 | - |
| Aqua | ad 100 | ad 100 | ad 100 | ad 100 | ad 100 | ad 100 |

### 2. Wasser-in-Oel-Emulsion

| | 1 | 2 | 3 | 4 | 5 |
|---|---|---|---|---|---|
| Lameform TGI | 3,0 | 3,0 | 3,0 | 3,0 | 3,0 |
| PEG-45/Dodecyl Glycol Copolymer | 0,5 | 0,5 | 0,5 | 0,5 | 0,5 |
| Microcrystalline Wax | 3,0 | 3,0 | 3,0 | 3,0 | 3,0 |
| Bis-Diglyceryl Polyacyladipate-2 | 1,0 | 1,0 | 1,0 | 1,0 | 1,0 |
| Paraffinöl | 8,0 | 8,0 | 8,0 | 8,0 | 8,0 |
| Vaseline | 2,0 | 2,0 | 2,0 | 2,0 | 2,0 |
| Vitamin-E-acetat | 2,0 | 2,0 | 2,0 | 2,0 | 2,0 |
| Methylparaben | 0,3 | 0,3 | 0,3 | 0,3 | 0,3 |
| Propylparaben | 0,3 | 0,3 | 0,3 | 0,3 | 0,3 |
| Isopropylisostearate | 8,0 | 8,0 | 8,0 | 8,0 | 8,0 |
| Glycerol | 5,0 | 3,0 | 3,0 | 3,0 | 3,0 |
| Mg-Sulfate | 0,5 | 0,5 | 0,5 | 0,5 | 0,5 |
| Caomint | 1,0 | - | 2,0 | 2,0 | 2,0 |
| Calmosensine | 1,0 | 2,0 | - | - | - |
| Milchsäure 80%ig | 0,5 | 0,56 | 0,56 | 0,56 | 0,56 |
| Omega-CH-Aktivator | 1,0 | - | - | - | - |
| Panthenol | 0,5 | 1,0 | 0,5 | 0,5 | 0,5 |
| Ultrasomes | 2,0 | - | 1,0 | - | - |
| Propylene Glycol | 0,5 | - | - | - | - |
| Thymidin-Dinucleotid | 0,05 | 0,1 | - | - | - |
| Carnitin | - | 0,5 | 0,5 | - | - |
| Gluconsäure | - | - | - | - | 1,5 |
| Äpfelsäure | - | - | - | 2,0 | 0,5 |
| α-Glucosylrutin | 0,05 | - | - | 0,05 | - |
| Sepilift DPHP | 1,00 | - | - | - | - |
| Exsy Algine | - | - | 2,00 | - | - |
| Ridulisse C | 0,40 | 0,40 | 0,40 | 0,50 | 0,70 |
| Phytokine | 1,50 | 2,00 | 1,50 | 1,50 | 1,50 |
| Liftiline | 3,00 | 3,00 | 3,00 | 2,50 | 2,50 |
| Sodium ascorbylphosphat | - | -- | - | - | 1,00 |
| Kombuchka | - | 3,00 | - | - | - |
| Glistin | - | 2,00 | - | - | - |
| Matrixyl 3000 | 2,00 | - | 2,00 | 2,00 | - |
| Deliner | - | - | 1,00 | - | - |
| Olea europ. Fol extr. S. sicc. | - | 0,10 | - | - | 0,10 |
| Parfum | 0,2 | 0,2 | 0,2 | 0,2 | 0,2 |
| Aqua | ad 100 | ad 100 | ad 100 | ad 100 | ad 100 |

### 3. Wasser-in-Silicon-Emulsion

| | 1 | 2 | 3 | 4 | 5 | 6 |
|---|---|---|---|---|---|---|
| Dimethicone | 2,50 | 2,50 | 2,50 | 2,50 | 2,50 | 2,50 |
| Cyclomethicone | 25,00 | 25,00 | 25,00 | 25,00 | 25,00 | 25,00 |
| Cetyl Dimethicone Copolyol | 2,00 | 2,00 | 2,00 | 2,00 | 2,00 | 2,00 |
| Sodium Chloride | 2,00 | 2,00 | 2,00 | 2,00 | 2,00 | 2,00 |
| Sodium Benzoate | - | 0,20 | 0,20 | - | - | - |
| Perfume | 0,30 | 0,30 | 0,30 | 0,30 | 0,30 | 0,30 |
| Methylparaben | - | - | - | 0,20 | 0,20 | 0,20 |
| Propylparaben | - | - | - | 0,20 | 0,20 | 0,20 |
| Symdiol 68 | 0,30 | 0,50 | 0,30 | - | - | - |
| Phenoxyethanol | 0,50 | | 0,40 | 0,40 | 0,40 | 0,40 |
| Glycerol | 5,00 | 2,50 | 5,00 | 5,00 | 2,50 | 5,00 |
| Caomint | 1,00 | - | 2,00 | 1,00 | - | 2,00 |
| Calmosensine | 1,00 | 2,00 | - | 1,00 | 2,00 | - |
| Naringin | - | - | - | 0,02 | - | - |
| Soy Protein Isolate | - | - | - | - | 1,00 | 1,00 |
| Ederline L | 3,00 | 1,00 | - | - | - | - |
| Sepivinol R | - | - | 1,00 | - | 1,00 | - |
| Coenzym Q 10 | 0,01 | - | - | - | 0,02 | - |
| Crodarom Chardonnay | 0,20 | - | - | - | 0,20 | - |
| Lipobelle Soyaglycone | - | 0,50 | - | - | - | - |
| Sambucus AO | - | - | 1,00 | - | - | - |
| Lotus germ extract BG | - | - | - | 0,15 | - | 0,50 |
| Ederline L | 1,00 | - | - | 2,00 | 2,00 | - |
| Matrixy13000 | 2,00 | - | 1,00 | 1,00 | - | - |
| SYN COLL | - | 1,00 | - | - | - | 1,00 |
| Ridulisse C | 0,50 | 1,00 | 1,00 | 0,50 | 1,00 | 0,50 |
| Phytokine | 1,50 | 2,00 | 1,50 | 1,50 | 1,50 | 1,50 |
| Liftiline | 3,00 | 3,00 | 3,00 | 2,50 | 2,50 | 2,50 |
| Aqua | ad 100 | ad 100 | ad 100 | ad 100 | ad 100 | ad 100 |

### 3. Reinigungszubereitungen

### 1. Gesichtswasser-Zubereitungen:

| | 1 | 2 | 3 |
|---|---|---|---|
| Dipropylenglycol | 10,00 | 10,00 | 10,00 |
| Chlorhexidindigluconate | 1,00 | 1,00 | 1,00 |
| Poloxamer-184 | 3,00 | 3,00 | 3,00 |
| Panthenol | 0,50 | 0,50 | 0,50 |
| Hydagen CMF | 3,00 | 3,00 | 3,00 |
| PEG-40 Hydrogenated Castor Oil Trideceth 9 / Propylene Glycol | / 0,50 | 0,50 | 0,50 |
| Chlorella Vulgaris Extract | 0,50 | 0,50 | 0,50 |
| Biopeptide CL | 1,00 | 1,00 | - |
| Ridulisse C | 0,50 | 1,00 | 1,00 |
| Phytokine | 1,50 | 2,00 | 1,50 |
| Liftiline | 3,00 | 3,00 | 3,00 |
| Perfume | 0,20 | 0,20 | 0,20 |
| Aqua | ad 100 | ad 100 | ad 100 |

### 2. Reinigungsgel:

| | 1 | 2 | 3 |
|---|---|---|---|
| Carbomer | 1,40 | 1,40 | 1,40 |
| Sorbitol | 2,10 | 2,10 | 2,10 |
| Sodium Benzoate | 0,40 | 0,40 | 0,40 |
| Plantaren^{®} 1200 | 7,50 | 7,50 | 7,50 |
| Dehyton^{®} K | 3,40 | 3,40 | 3,40 |
| Texapon^{®} SB 3 | 5,00 | 5,00 | 5,00 |
| Cetiol^{®} HE | 0,50 | 0,50 | 0,50 |
| Lamesoft^{®} PO 65 | 5,00 | 5,00 | 5,00 |
| Controx KS | 0,05 | 0,05 | 0,05 |
| Sodium PCA | 1,60 | 1,60 | |
| Pantolactone | 1,00 | 1,00 | |
| Tetrasodium EDTA | 0,25 | 0,25 | 0,25 |
| Sodium Lactate | 1,80 | | |
| Ridulisse C | 0,50 | 1,00 | 1,00 |
| Phytokine | 1,50 | 2,00 | 1,50 |
| Liftiline | 3,00 | 3,00 | 3,00 |
| Acnacidol PG | 0,10 | 0,50 | - |
| Exsy-Algine | 2,00 | - | - |
| Glistin | - | 0,50 | - |
| Keratec pep | - | - | 0,50 |
| Kombuchka | - | 0,75 | - |
| Matrixyl 3000 | - | 0,50 | - |
| Olea europ. Fol extr. S. sicc. | 0,10 | - | 0,10 |
| Perfume | 0,40 | 0,40 | 0,40 |
| Aqua | ad 100 | ad 100 | ad 100 |

### 2-Phasen-Wellness-Massagefluid für Körper und Kopfhaut

| | | |
|---|---|---|
| | | |
| Allantoin | 0,100000 | 0,100000 |
| D&C Green No. 5 | 0,000800 | 0,000800 |
| Caomint | 1,000000 | - |
| Calmosensine | - | 1,000000 |
| Milchsäure 80% DAB | 0,100000 | 0,100000 |
| Dinatriumphosphat wasserfrei | 0,100000 | 0,100000 |
| D-Panthenol 75 % | 0,100000 | 0,100000 |
| Parfum | 0,150000 | 0,150000 |
| Brij 30 | 0,500000 | 0,500000 |
| Trisiloxane Fluid 1 cs | 20,000000 | 20,000000 |
| d,1-alpha-Bisabolol | 0,100000 | 0,100000 |
| Kombuchka | 1,000000 | 2,000000 |
| Glistin | 0,250000 | - |
| Retinol | - | 0,100000 |
| Ridulisse C | 0,500000 | 1,000000 |
| Phytokine | 1,500000 | 2,000000 |
| Liftiline | 3,000000 | 3,000000 |
| Olea europ. Fol extr. S. sicc. | 0,100000 | - |
| Ethanol 96 % DEP vergällt | 25,000000 | 25,000000 |
| Wasser, vollentsalzt | ad 100,000000 | ad 100,000000 |

### Beispiele für Zusammensetzungen zum Tränken von Tüchern

| | Bestandteile | 1 | 2 | 3 | 4 |
|---|---|---|---|---|---|
| Phase 1 | PEG-40 Hydrogenated Castor Oil | 0,1 | 0,1 | 0,1 | - |
| | Trideceth-9 | 0,1 | 0,1 | 0,1 | - |
| | Parfum | 0,3 | 0,1 | 0,1 | 0,1 |
| Phase 2 | Hexyllaurat | 2,0 | - | - | - |
| | Dicaprylylcarbonat | - | - | 2,0 | - |
| | 1-(4-tert.-Butylphenyl)-3-(4'methoxyphenyl)propan-1,3-dion (Parsol^{®} 1789) | - | - | 1,0 | - |
| | 3-(4'-Methylbenzyliden)-D,L-campher | - | - | 1.5 | - |
| | Dipropylenglycol | - | 2,0 | - | - |
| | Dihydroxyaceton | - | 1,0 | - | - |
| Phase 3 | Panthenol | 0,5 | - | - | - |
| | Citronensäure (Monohydrat) | 1,5 | 1,5 | 1,5 | 1,5 |
| | Trinatriumcitrat (Dihydrat) | 2,0 | 2,0 | 2,0 | 2,0 |
| | Natriumsalicylat | 0,45 | - | - | - |
| | Decylglucosid | - | - | - | 2,0 |
| | Pemulen TR 1 | - | - | - | 0,2 |
| | Kombuchka | 3,00 | 3,00 | 1,00 | 3,00 |
| | Glistin | 2,00 | - | 0,50 | - |
| | Ridulisse C | 0,50 | 0,50 | 0,50 | 1,00 |
| | Liftiline | 2,00 | 1,00 | 2,00 | 3,00 |
| | Phytokine | 1,50 | 1,50 | 2,00 | 1,50 |
| | Wasser | ad 100 | ad 100 | ad 100 | ad 100 |

Mit diesen Tränkzusammensetzungen wurden verschiedene Viskose-Vliesstoffe ausgerüstet. Es wurden gelochte, ungelochte, genoppte, einlagige, zweilagige und dreilagige Vliese ausgerüstet. Die Tücher wurden sowohl als feuchte Tücher als auch als trockene Tücher (das heißt, nach dem Ausrüsten wurden die Tücher bis auf einen Restwassergehalt kleiner 10 Gew.-%, bevorzugt kleiner 5 Gew.-%, getrocknet) verwendet.
Beispiel 1 stellt eine Tränkzusammensetzung für ein Lotions- und Make up-Entferner-Tuch dar.
Beispiel 2 stellt eine Tränkzusammensetzung für ein Selbstbräuner-Tuch dar.
Beispiel 3 stellt eine Tränkzusammensetzung für ein Sonnenschutz-Tuch dar.
Beispiel 4 stellt eine Tränkzusammensetzung für ein Gesichtsreinigungs-Tuch dar.

### Hautaufhellende Anti Age-Matrixpflaster

| | 1 | 2 | 3 | 4 | 5 |
|---|---|---|---|---|---|
| DURO-TAK^{®} 387-2516 | 76 | 76 | 76 | 76 | 76 |
| Natriumascorbylphosphat | 3 | 2 | 3 | - | 2 |
| Magnesiumascorbylphosphat | - | - | - | 2 | 1 |
| Vitamin B6 | 0,05 | 0,05 | - | 0,05 | - |
| Naringin | 0,1 | - | - | - | - |
| Calmosensine | 0,5 | 2 | - | 0,5 | - |
| Herbasol^{®} Destillat Grüner Tee | - | - | 2 | - | - |
| Aloe Vera Gel | 1 | - | 1 | - | - |
| Caomint | 1,0 | - | 2,0 | 2,0 | 2,0 |
| Tioveil^{®}-AQ-N | 2 | 2 | - | - | 2 |
| Eusolex^{®} OCR | 1 | - | 1 | 1 | 1 |
| Propylenglycolmonooleat | 5 | 5 | - | - | - |
| Tween^{®}-80 | - | - | 5 | 3 | 5 |
| Natriumcitrat | 0,1 | 0,1 | 0,1 | 0,1 | 0,1 |
| Controx^{®} KS | 0,05 | 0,05 | 0,05 | 0,05 | 0,05 |
| Natrulon RC 50DG | 1,00 | - | 1,00 | - | - |
| Pearl Protein Extract | - | 2,00 | - | - | 2,00 |
| Glistin | - | - | 2,00 | 2,00 | - |
| Ridulisse C | 0,50 | 1,00 | 0,50 | 0,50 | 0,50 |
| Liftiline | 3,00 | 3,00 | 1,00 | 1,00 | 3,00 |
| Phytokine | 2,00 | 1,50 | 1,50 | 1,00 | 2,00 |
| Wasser | ad 100 | ad 100 | ad 100 | ad 100 | ad 100 |

### Hautberuhigende Anti-Age-Reservoirpflaster

| Bestandteile des Wirkstoffreservoirs | 1 | 2 |
|---|---|---|
| Caomint | 2 | 2 |
| Calmosensine | 0,5 | - |
| Ederline H | 1,0 | 1,0 |
| Carbopol^{®} 980 | 0,5 | - |
| Pemulen^{®}TR 1 | - | 0,5 |
| NaOH | 0,1 | 0,1 |
| Titandioxid | 0,2 | 0,2 |
| Ethanol | 1,0 | 1,0 |
| Polyvinylalkohol | 2,0 | 1,0 |
| Carboxymethylcellulose | 1,0 | 0,5 |
| Glycerin | 10 | 20 |
| Sorbitol | 7,0 | 5,0 |
| 1,3-Butandiol | 3,0 | 3,0 |
| Tween^{®}-80 | 0,05 | 0,05 |
| Paraffinöl | 5,0 | 2,0 |
| Natriumcitrat | 0,1 | 0,1 |
| Controx KS | 0,05 | 0,05 |
| Kombuchka | 3,00 | - |
| Glistin | 2,00 | - |
| Natrulon RC 50 DG | - | 1,00 |
| Olea europ. Fol extr. S. sicc. | - | 0,10 |
| Crodarom Chardonnay | - | 1,00 |
| Phytokine | 2,00 | 2,00 |
| Ridulisse C | 0,50 | 0,40 |
| Liftiline | 3,00 | 3,00 |
| Wasser | ad 100 | ad 100 |

Die Bestandteile der Wirkstoffreservoir-Rezepturen Nr. 1 bzw. 2 wurden miteinander vermischt, so dass eine gelartige Masse entstand. Mit dieser Masse wurden Pflasterträger beschichtet, so dass sogenannte Reservoirpflaster erhalten wurden. Diese Pflaster werden sowohl auf die trockene Haut als auch auf die angefeuchtete Haut gelegt und verbleiben dort für eine Zeit von wenigen Sekunden bis einigen Stunden.

### Liste der verwendeten Rohstoffe

| Handelsname | INCI-Bezeichnung | Lieferant/Hersteller |
|---|---|---|
| | | |
| Acnacidol PG | Sebacic acid, 10-hydroxydecanoic acid, 1,10-decanediol | Vincience |
| Ajidew^{®} NL 50 | Sodium PCA | AJINOMOTO |
| Baysilone-Öl M 350 | Dimethicone | GE Bayer Silicones |
| Biodocarb^{®} | lodopropynyl Butylcarbamate | MILKER & GRÜNING |
| Biopeptide CL | N-Palmitoyl-Gly-His-Lys | Sederma |
| Biopeptide EL | N-Palmitoyl-Val-Gly-Val-Ala-Pro-Gly | Sederma |
| Brij 30 | Laureth-4 | Uniqema |
| Calmosensine | BUTYLENE GLYCOL, WATER, LAURETH-3, HYDROXYETHYLCELLULOSE, Acetyl Dipeptide-1 Cetylester | Sederma |
| Caomint | Propylene Glycol, Aqua, Mentha piperita, Theobroma cacao | Solabia |
| Carbopol 980 | Carbomer | Noveon |
| Carbopol ETD 2020 Cetiol HE | Acrylates/C10-30 Alkyl Acrylate Crosspolymer | Noveon |
| | PEG-7 Glyceryl Cocoate | Cognis |
| Cetiol^{®} PGL | Hexyldecanol, Hexyldecyl Laurate | Cognis |
| Controx KS | Tocopherol, Hydrogenated Palm Glycerides Citrate | Cognis |
| Cosmedia Guar^{®} C 261 | Guar Hydroxypropyl Trimonium Chloride | Cognis |
| Crodarom Chardonnay L | Propylene Glycol, Water, Vitis Vinifera (Grape) Seed Extract | Croda |
| Cutina CBS | Glyceryl Stearate / Cetearyl Alcohol / Cetyl Palmitate / Cocoglycerides | Cognis |
| Cutina GMS | Glyceryl Stearate (Mschung aus Glyceryl-mono- und distearat) | Cognis |
| Cutina MD | Glyceryl Stearate | Cognis |
| DC^{®} 190 | Dimethicone | Dow Corning |
| Dehyton K | AQUA (WATER), COCAMIDOPROPYL BETAINE (29 - 32 Gew.-%) | Cognis |
| Deliner | Zea Mays (Corn) Kernel Extract, Butylene Glycol, Xanthan Gum | Coletica |
| DERMOSOFT OCTIOL | 1,2-Octandiol | Dr. Straetmans |
| Dow Corning 1501 Fluid | Cyclomethicone (85- 86 Gew.-%), Dimethiconol (14-15 Gew.-%) | Dow Corning |
| Dow Corning 200 (0,65 cSt) | Dimethicone | Dow Corning |
| Dow Corning 9040 | Cyclomethicone/ Dimethicone Crosspolymer | Dow Corning |
| Dow Corning^{®}200 Fluid, 5 cSt. | Dimethicone | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinylpyrrolidon | National Starch and Chemical |
| Ederline H | PEG-40 Hydrogenated Castor Oil, PPG-2-Ceteareth-9, Pyrus Malus (Apple) Fruit Extract | Seporga |
| Ederline L | Hexyldecanol, Pyrus Malus (Apple) Fruit Extract | Seporga |
| EMULGADE^{®} SE | Glyceryl Stearate, Ceteareth-20, Ceteareth-12, Cetearyl Alcohol, Cetyl Palmitate | Cognis |
| Eumulgin B 2 | Ceteareth-20 | Cognis |
| Eumulgin B 3 | Ceteareth-30 | Cognis |
| Eumulgin 05 | Oleth-5 | Cognis |
| Eusolex^{®} OCR | Octocrylene | Merck KGaA |
| Exsy Algine | ACETYL CITRULL AMIDO ARGININE | Exsymol |
| Fucogel 1000 | Biosaccharide Gum-1 | Solabia |
| Germall^{®} 115 | Imidazolidinyl Urea | Sutton Laboratories |
| Glistin | Wasser, L-Glutamylaminoethyl indole | Exsymol |
| Glucamate^{®}DOE 120 | PEG-120 Methyl Glucose Dioleate | AMERCHOL |
| Hibiscin^{®} HP-LS-9198 | Water, Hibiscus esculentus Seed Extract, Phenoxyethanol | Laboratoires Serobiologiques |
| Honeyquat^{®} 50 | Hydroxypropyltrimonium Honey | BROOKS |
| Hydagen CMF | Chitosan Glycolate | Cognis |
| Hydrovance | Wasser, Bis-N,N'-(2-Hydroxyethyl)harnstoff, Harnstoff, Ammoniumlactat | National Starch |
| Jaguar HP 105 | HYDROXYPROPYL GUAR | Rhodia |
| Keltrol SF | Xanthan Gum | Kelco |
| Keratec Pep | Water, Hydrolyzed Keratin | Croda |
| Kombuchka | Saccharomyces/Xylinum/Black Tea Ferment, Glycerin, Hydroxyethylcellulose | Sederma |
| Lameform TGI | Polyglyceryl-3 Diisostearate | Cognis |
| Lamesoft^{®} PO 65 | Coco-Glucoside, Glyceryl Oleate, Water | Cognis |
| LANETTE^{®} O | Cetearyl Alcohol | Cognis |
| LANETTE^{®} 22 | Behenyl Alcohol | Cognis |
| Liftiline | Aqua, Hydrolyzed Wheat Protein (7-10 Gew.-% Aktivsubstanz) | Silab |
| Lipobelle Soyaglycone | Alcohol, Polysorbate 80, Soy Isoflavones | Mibelle AG Cosmetics |
| Lipoid S 75-3 | Aqua, Lecithine | Lipoid GmbH |
| Lotus Germ Extract | Water, Butylene Glycol, Nelumbo Nucifera Germ Extract | Maruzen |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Montanov L | C14-22 Alcohols/C12-20 Alkyl Glucoside | Seppic |
| Myritol 318 | Caprylic/Capric Triglyceride | Cognis |
| MYRITOL^{®} 312 | Caprylic/Capric Triglyceride | Cognis |
| MYRITOL^{®} PC | PROPYLENE GLYCOL DICAPRYLATE/DICAPRATE | Cognis |
| Natrulon RC 50 DG | WATER, CARNITINE, POLYGLYCERIN-10 | Lonza |
| Neutrol TE | TETRAHYDROXYPROPYL ETHYLENEDIAMINE | BASF |
| Novata AB | Cocoglycerides | Cognis |
| Olea europ. Fol extr. S. sicc. | Olea Europea (Olive) Leaf Extract | Fruitarom |
| Omega-CH-Aktivator | Tripeptide-1 | GfN |
| Pearl Protein Extract | Aqua, Hydrolyzed Conchiolin Protein | Maruzen |
| Pemulen TR 1 | Acrylates/C10-30 Alkyl Acrylate Crosspolymer | Noveon |
| Phenonip^{®} | Phenoxyethanol, Methylparaben, Ethylparaben, Propylparaben, Butylparaben, ca. 28 % Aktivsubstanz | NIPA |
| Photosomes | Aqua, Lecithine, Plancton Extract | AGI Dermatics |
| Phytodermin | SOYBEAN PROTEIN GLYCINE SOJA (Linne) | CLR Chem. Laboratorium Kurt Richter |
| Phytokine | Water, HYDROLYZED SOY PROTEIN (0,4 - 0,8 Gew.-% Aktivsubstanz) | Coletica |
| Plantacare^{®} 818 UP | Coco Glucoside, ca. 50 % Aktivsubstanz | Cognis |
| Plantaren^{®} 1200 | Lauryl Glucoside, ca. 50 % Aktivsubstanz | Cognis |
| Plantaren^{®} 2000 | Decyl Glucoside, ca. 50 % Aktivsubstanz | Cognis |
| Polymer JR 400^{®} | Polyquaternium-10 | UNION CARBIDE |
| Ridulisse C | Water, HYDROLYZED SOY PROTEIN (3-5 Gew.-% Aktivsubstanz) | Silab |
| Sambucus AO | Water, Glycerin, Sambucus Nigra Flower Extract | Alpaflor/Centerchem |
| Sensiva^{®} SC 50 | 2-Ethylhexylglycerinether | Schülke & Mayr |
| Sepigel^{®}305 | Polyacrylamide, C₁₃-C₁₄ Isoparaffin, Laureth-7 | SEPPIC |
| Sepivinol R | Wine Extract (Polyphenolreicher Extrakt aus Rotwein) | Seppic |
| Sepilift DPHP | Dipalmitoyl Hydroxyproline | Seppic |
| Silymarin Phytosome | Silybum Marianum Extract and Phospholipids | Indena SpA |
| Simulgel NS | Hydroxyethyl Acrylate / Sodium Acryloyldimethyl Taurate Copolymer / Squalane / Polysorbate 60 | Seppic |
| Soy Protein Isolate | | Protein Technology International |
| Stenol 16/18 | Cetearylalkohol | Cognis |
| Surfadone LP 300 | N-Laurylpyrrolidon | ISP |
| Symdiol 68 | 1,2-Octandiol, 1,2-Hexandiol | Symrise |
| SYN^{®}-COLL | Water, Palmitoyl-Lys-Val-Lys | Pentapharm |
| Texapon^{®} NSO | Sodium Laureth Sulfate, Aqua (ca. 28 % Aktivsubstanz) | Cognis |
| Texapon^{®} SB 3 | Aqua, Disodium Laureth Sulfosuccinate, (ca. 40 % Aktivsubstanz), Citric Acid | Cognis |
| Tioveil^{®}-AQ-N | Cl 77891 (Titanium Dioxide),Alumina, Silica, Sodium Polyacrylate | Uniqema (Tioxide Specialties) |
| Tween^{®}-80 | Polysorbate-80 | |
| Ultrahold 8 | ACRYLATES/t-BUTYLACRYLAMIDE COPOLYMER | BASF |
| Ultrasomes | Aqua, Lecithine, Micrococcus Luteus Extract | AGI Dermatics |
| Uvinul MS 40 | BENZOPHENONE-4 | BASF |
| Vegetal Filling Spheres | CAPRYLIC/CAPRIC TRIGLYCERIDE, SILICA DIMETHYL SILYLATE, HYDRO-LYZED WHEAT PROTEIN, BUTYLENE GLYCOL, PHENOXYETHANOL, METHYL-PARABEN, ETHYLPARABEN, PROPYL-PARABEN, BUTYLPARABEN, ISOBUTYL-PARABEN | Coletica |

## Patentansprüche

1. Kosmetische oder dermatologische Zusammensetzung zur topischen Behandlung der Haut, enthaltend in einem geeigneten kosmetischen oder dermatologischen Träger
a) mindestens ein Sojaproteinhydrolysat mit einem mittleren Molekulargewicht im Bereich von 1200 - 1800 Dalton, bevorzugt im Bereich von 1400 - 1700 Dalton,
b) mindestens ein Sojaproteinhydrolysat mit einem mittleren Molekulargewicht im Bereich von 600 - 1000 Dalton, bevorzugt 800 Dalton, und
c) mindestens ein Weizenproteinhydrolysat.

2. Kosmetische oder dermatologische Zusammensetzung gemäß Anspruch 1, **dadurch gekennzeichnet, dass** weiterhin mindestens ein die Kollagensynthese stimulierender Wirkstoff enthalten ist, der ausgewählt ist aus
- Hydroxyprolin, das mit zwei, bevorzugt linearen, C₂-C₂₂-Fettsäureresten N-acyliert und verestert ist, insbesondere Dipalmitoylhydroxyprolin,
- den Tripeptiden Gly-His-Lys, Lys-Val-Lys, Lys-Val-Dab, Lys-Phe-Lys, Lys-Ile-Lys, Dab-Val-Lys, Lys-Val-Orn, Lys-Val-Dap, Dap-Val-Lys und Gly-His-Arg,
- den Tetrapeptiden Gly-Gln-Pro-Arg (Rigin), Gly-Gln-Arg-Pro, Val-Val-Arg-Pro, Rigin-Analoga sowie ALAMCAT-Tetrapeptiden,
- dem Pentapeptid Lys-Thr-Thr-Lys-Ser,
- den Hexapeptiden Val-Gly-Val-Ala-Pro-Gly, Ala-Asp-Leu-Lys-Pro-Thr (Hexapeptide-3), Hexapeptide-4, Hexapeptide-5, Hexapeptide-6, Hexapeptide-8, Hexapeptide-9 und Hexapeptide-10,
- den mit mindestens einer, bevorzugt linearen, C₂-C₂₂-Fettsäure N-acylierten und/oder veresterten Derivaten der genannten Tri-, Tetra-, Penta- und Hexapeptide, insbesondere N-Palmitoyl-Gly-His-Lys, N-Palmitoyl-Lys-Val-Lys, N-Myristoyl-Gly-His-Arg, N-Palmitoyl-Gly-Gln-Pro-Arg, N-Palmitoyl-Lys-Thr-Thr-Lys-Ser, N-Palmitoyl-Val-Gly-Val-Ala-Pro-Gly, Acetyl-Hexapeptide-3, Myristoyl Hexapeptide-5, Myristoyl Hexapeptide-8 sowie Mischungen aus N-Palmitoyl-Gly-His-Lys und N-Palmitoyl-Gly-Gln-Pro-Arg,
- Retinoiden, insbesondere Retinol und C₂-C₂₂-Fettsäureestern des Retinols,
- Saccharomyces/Xylinum/Black Tea Ferment,
- Olivenblattextrakten (Olea Europaea (Olive) Leaf Extract),
- Oleanolsäure,
- Oleanol,
- Extrakten aus Haferkörnern (Avena Sativa (Oat) Kernel Extract),
- liposomenverkapselten Grüntee-Extrakten (Camellia Sinensis),
- Kreatin,
- sowie Mischungen der vorgenannten Substanzen.

3. Kosmetische oder dermatologische Zusammensetzung gemäß einem der vorstehenden Ansprüche, **dadurch gekennzeichnet, dass** weiterhin mindestens ein Wirkstoff, der die Interaktion zwischen der extrazellulären Matrix und den Fibroblasten erhöht und/oder verbessert, enthalten ist, der ausgewählt ist aus
- mit Kokosfettsäuren N-acylierten und/oder veresterten Sojaproteinhydrolysaten in Form ihrer Alkalimetallsalze, bevorzugt in Form der Kaliumsalze,
- Ascorbinsäure, den Estern der Ascorbinsäure mit anorganischen und/oder organischen Säuren, den Ethern der Ascorbinsäure mit Mono-, Oligo- und Polysacchariden sowie den physiologisch verträglichen Salzen dieser Komponenten,
- Apfelkernextrakten (Pyrus Malus (Apple) Fruit Extract),
- Lotuskeim-Extrakten (Nelumbo Nucifera Germ Extract),
- Extrakten aus Maiskörnern (Zea Mays (Corn) Kernel Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Mischungen aus mindestens einem Extrakt aus Kakaobohnen (Theobroma cacao) und mindestens einem Extrakt aus den Blättern der Pfefferminze (Mentha piperita),
- Hydroxystilbenen und deren Estern, insbesondere Resveratrol und/oder Resveratrolmono-, -di- und -triphosphorsäureestern und deren Salzen,
- Keratinhydrolysaten, insbesondere Wollkeratinhydrolysaten,
- Conchiolinhydrolysaten,
- dem Dipeptid L-Citrullyl-L-arginin und seinen mit mindestens einer, bevorzugt linearen, C₂-C₂₂-Fettsäure N-acylierten und/oder veresterten Derivaten, bevorzugt Acetyl Citrull Amido Arginine,
- Carnitin,
- Hypotaurin,
- L-Glutamylaminoethyl-indol,
- Isoflavonoiden und Isoflavonoid-reichen Pflanzenextrakten,
- Dihydroquercetin (= Taxifolin),
- sowie Mischungen hiervon.

4. Kosmetische oder dermatologische Zusammensetzung gemäß einem der vorstehenden Ansprüche, enthaltend mindestens einen weiteren kosmetischen Wirkstoff, ausgewählt aus:
a) Monomeren und Oligomeren von Aminosäuren, N-C₂-C₂₄-Acylaminosäuren, den Estern und/oder den physiologisch verträglichen Metallsalzen dieser Substanzen, die nicht die Kollagensynthese stimulieren und nicht die Interaktion zwischen der extrazellulären Matrix und den Fibroblasten erhöhen und/oder verbessern,
b) DNA-Reparaturenzymen,
c) DNA- oder RNA-Oligonucleotiden,
d) Vitaminen, Provitaminen und Vitaminvorstufen der Gruppen B, E, H und K und den Estern der vorgenannten Substanzen,
e) α-Hydroxycarbonsäuren, α-Ketocarbonsäuren, β-Hydroxycarbonsäuren und deren Ester-, Lacton- und/oder Salzform,
f) Flavonoiden und Flavonoid-reichen Pflanzenextrakten, die nicht die Kollagensynthese stimulieren und nicht die Interaktion zwischen der extrazellulären Matrix und den Fibroblasten erhöhen und/oder verbessern,
g) Ubichinon und Ubichinol sowie deren Derivaten,
h) Silymarin,
i) Ectoin,
j) anorganischen und organischen UV-Filtersubstanzen,
k) selbstbräunenden Wirkstoffen,
l) hautberuhigenden Wirkstoffen,
m) feuchtigkeitsspendenden Wirkstoffen,
n) sebumregulierenden Wirkstoffen,
o) sowie Mischungen dieser Wirkstoffe a) - o).

5. Kosmetische oder dermatologische Zusammensetzung gemäß einem der vorstehenden Ansprüche, **dadurch gekennzeichnet, dass** die Monomeren von Aminosäuren, N-C₂-C₂₄-Acylaminosäuren und/oder den physiologisch verträglichen Metallsalzen dieser Substanzen, die nicht die Kollagensynthese stimulieren und nicht die Interaktion zwischen der extrazellulären Matrix und den Fibroblasten erhöhen und/oder verbessern, ausgewählt sind aus
- Alanin, Arginin, Asparagin, Asparaginsäure, Canavanin, Citrullin, Cystein, Cystin, Desmosin, Glutamin, Glutaminsäure, Glycin, Histidin, Hydroxylysin, Isodesmosin, Isoleucin, Leucin, Lysin, Methionin, Ornithin, Phenylalanin, Prolin, Pyroglutaminsäure, Sarcosin, Serin, Taurin, Threonin, Thyroxin, Tryptophan, Tyrosin, Valin, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat,
- sowie Mischungen dieser Substanzen.

6. Kosmetische oder dermatologische Zusammensetzung gemäß einem der vorstehenden Ansprüche, **dadurch gekennzeichnet, dass** mindestens ein nichtionischer Emulgator mit einem HLB-Wert von 8 und darunter der allgemeinen Formel R¹ - O - R², in der R¹ eine primäre lineare Alkyl-, Alkenyl- oder Acylgruppe mit 20 - 30 C-Atomen, bevorzugt einen Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2-5 Hydroxylgruppen, bevorzugt Wasserstoff, darstellt, bevorzugt Behenylalkohol, enthalten ist.

7. Kosmetische oder dermatologische Zusammensetzung gemäß einem der vorstehenden Ansprüche, **dadurch gekennzeichnet, dass** sie in Form einer lamellare Strukturen aufweisenden ÖI-in-Wasser-Emulsion vorliegt.

8. Verwendung einer Kombination von
(a) mindestens einem Sojaproteinhydrolysat mit einem mittleren Molekulargewicht im Bereich von 1200 - 1800 Dalton, bevorzugt im Bereich von 1400 - 1700 Dalton,
(b) mindestens einem Sojaproteinhydrolysat mit einem mittleren Molekulargewicht im Bereich von 600 -1000 Dalton, bevorzugt 800 Dalton, und
(c) mindestens einem Weizenproteinhydrolysat
zur Herstellung einer kosmetischen oder dermatologischen Zusammensetzung zur Behandlung lichtgeschädigter und/ oder lichtgealterter und/oder intrinsisch und/oder extrinsisch gealterter Haut, insbesondere zur Antifaltenbehandlung.

9. Verwendung einer Kombination von
(a) mindestens einem Sojaproteinhydrolysat mit einem mittleren Molekulargewicht im Bereich von 1200 - 1800 Dalton, bevorzugt im Bereich von 1400 - 1700 Dalton,
(b) mindestens einem Sojaproteinhydrolysat mit einem mittleren Molekulargewicht im Bereich von 600 - 1000 Dalton, bevorzugt 800 Dalton, und
(c) mindestens einem Weizenproteinhydrolysat
zur Herstellung einer kosmetischen oder dermatologischen Zusammensetzung zur Steigerung der Hautfestigkeit und/oder zur Verbesserung der mechanischen Stabilität der Haut und/oder zur Steigerung der Hautelastizität und/oder der Hautstraffheit und/oder zur Verbesserung der Hautweichheit und Hautgeschmeidigkeit.

10. Verwendung einer Kombination von
(a) mindestens einem Sojaproteinhydrolysat mit einem mittleren Molekulargewicht im Bereich von 1200 - 1800 Dalton, bevorzugt im Bereich von 1400 - 1700 Dalton,
(b) mindestens einem Sojaproteinhydrolysat mit einem mittleren Molekulargewicht im Bereich von 600 -1000 Dalton, bevorzugt 800 Dalton, und
(c) mindestens einem Weizenproteinhydrolysat
zur Herstellung einer kosmetischen oder dermatologischen Zusammensetzung zur Glättung der Haut und/oder zur Verminderung von Falten, Fältchen und/oder feinen Linien und/oder zur Verbesserung des optischen Erscheinungsbildes der Haut.

11. Verwendung einer Kombination von
(a) mindestens einem Sojaproteinhydrolysat mit einem mittleren Molekulargewicht im Bereich von 1200 - 1800 Dalton, bevorzugt im Bereich von 1400 - 1700 Dalton,
(b) mindestens einem Sojaproteinhydrolysat mit einem mittleren Molekulargewicht im Bereich von 600 - 1000 Dalton, bevorzugt 800 Dalton, und
(c) mindestens einem Weizenproteinhydrolysat
zur Herstellung einer kosmetischen oder dermatologischen Zusammensetzung zum Schutz und/oder zur Prophylaxe vor extrinsischer Hautalterung und/oder zur Verzögerung der extrinsischen Hautalterung.

12. Verwendung einer Kombination von
(a) mindestens einem Sojaproteinhydrolysat mit einem mittleren Molekulargewicht im Bereich von 1200 - 1800 Dalton, bevorzugt im Bereich von 1400 - 1700 Dalton,
(b) mindestens einem Sojaproteinhydrolysat mit einem mittleren Molekulargewicht im Bereich von 600 - 1000 Dalton, bevorzugt 800 Dalton, und
(c) mindestens einem Weizenproteinhydrolysat
zur Herstellung einer kosmetischen oder dermatologischen Zusammensetzung zur Verzögerung der intrinsischen Hautalterung

13. Verwendung einer Kombination von
(a) mindestens einem Sojaproteinhydrolysat mit einem mittleren Molekulargewicht im Bereich von 1200 - 1800 Dalton, bevorzugt im Bereich von 1400 - 1700 Dalton,
(b) mindestens einem Sojaproteinhydrolysat mit einem mittleren Molekulargewicht im Bereich von 600 - 1000 Dalton, bevorzugt 800 Dalton, und
(c) mindestens einem Weizenproteinhydrolysat
zur Herstellung einer kosmetischen oder dermatologischen Zusammensetzung zur Behandlung von Altersflecken.

14. Verwendung einer Kombination von
(a) mindestens einem Sojaproteinhydrolysat mit einem mittleren Molekulargewicht im Bereich von 1200 -1800 Dalton, bevorzugt im Bereich von 1400 - 1700 Dalton,
(b) mindestens einem Sojaproteinhydrolysat mit einem mittleren Molekulargewicht im Bereich von 600-1000 Dalton, bevorzugt 800 Dalton, und
(c) mindestens einem Weizenproteinhydrolysat
zur Herstellung einer kosmetischen oder dermatologischen Zusammensetzung zur Aufrechterhaltung des normalen Proteinstoffwechsels der Haut.

15. Verwendung einer Kombination von
(a) mindestens einem Sojaproteinhydrolysat mit einem mittleren Molekulargewicht im Bereich von 1200 - 1800 Dalton, bevorzugt im Bereich von 1400 - 1700 Dalton,
(b) mindestens einem Sojaproteinhydrolysat mit einem mittleren Molekulargewicht im Bereich von 600 -1000 Dalton, bevorzugt 800 Dalton, und
(c) mindestens einem Weizenproteinhydrolysat
zur Herstellung einer kosmetischen oder dermatologischen Zusammensetzung zur Verminderung oxidativer Schäden in der Haut.

16. Verwendung einer Kombination von
(a) mindestens einem Sojaproteinhydrolysat mit einem mittleren Molekulargewicht im Bereich von 1200-1800 Dalton, bevorzugt im Bereich von 1400- 1700 Dalton,
(b) mindestens einem Sojaproteinhydrolysat mit einem mittleren Molekulargewicht im Bereich von 600 - 1000 Dalton, bevorzugt 800 Dalton, und
(c) mindestens einem Weizenproteinhydrolysat
zur Herstellung einer kosmetischen oder dermatologischen Zusammensetzung zur Verbesserung der Hautverträglichkeit von Hautbehandlungsmitteln mit Antiageing-Wirkung, insbesondere von Antifaltenmitteln

17. Verwendung nach einem der Ansprüche 8-16, **dadurch gekennzeichnet, dass** die Zusammensetzung mindestens einen weiteren Wirkstoff, wie in mindestens einem der Ansprüche 2 **-** 7 genannt, enthält.
